(19) Europäisches Patentamt
European Patent Office
Office européen des brevets

(11) **EP 3 775 260 B1**

(12) **EUROPEAN PATENT SPECIFICATION**

(45) Date of publication and mention
of the grant of the patent:
**24.12.2025   Bulletin 2025/52**

(21) Application number: **19776801.3**

(22) Date of filing: **27.03.2019**

(51) International Patent Classification (IPC):
**C12Q 1/68** (2018.01)

(52) Cooperative Patent Classification (CPC):
**C12Q 1/6886;** C12Q 2600/106; C12Q 2600/158;
Y02A 90/10

(86) International application number:
**PCT/US2019/024379**

(87) International publication number:
**WO 2019/191297 (03.10.2019 Gazette 2019/40)**

(54) **METHODS AND APPARATUS FOR QUANTIFYING PROTEIN ABUNDANCE IN TISSUES VIA CELL FREE RIBONUCLEIC ACIDS IN LIQUID BIOPSY**

VERFAHREN UND VORRICHTUNG ZUR QUANTIFIZIERUNG DER PROTEINABUNDANZ IN GEWEBE DURCH ZELLFREIE RIBONUKLEINSÄUREN BEI EINER FLÜSSIGBIOPSIE

PROCÉDÉS ET APPAREIL POUR QUANTIFIER L'ABONDANCE DE PROTÉINES DANS DES TISSUS PAR L'INTERMÉDIAIRE D'ACIDES RIBONUCLÉIQUES ACELLULAIRES DANS UNE BIOPSIE LIQUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2018   US 201862648984 P**

(43) Date of publication of application:
**17.02.2021   Bulletin 2021/07**

(73) Proprietor: **Certara USA, Inc.**
**Radnor, PA 19087 (US)**

(72) Inventors:
• **ROSTAMI-HODJEGAN, Amin**
  **Princeton, New Jersey 08540 (US)**
• **ACHOUR, Brahim**
  **Princeton, New Jersey 08540 (US)**
• **ROTHMAN, James Edward**
  **Princeton, New Jersey 08540 (US)**

(74) Representative: **Crease, Devanand John et al**
**Keltie LLP**
**No.1 London Bridge**
**London SE1 9BA (GB)**

(56) References cited:
WO-A1-02/00935          WO-A1-2017/156310
US-A1- 2009 162 842

• **VAN DER HAUWAERT CYNTHIA ET AL: "Expression profiles of genes involved in xenobiotic metabolism and disposition in human renal tissues and renal cell models", TOXICOLOGY AND APPLIED PHARMACOLOGY, ACADEMIC PRESS, AMSTERDAM, NL, vol. 279, no. 3, 15 July 2014 (2014-07-15), pages 409 - 418, XP029062863, ISSN: 0041-008X, DOI: 10.1016/J.TAAP.2014.07.007**
• **LAM JUSTINE L. ET AL: "Expression and Functional Analysis of Hepatic Cytochromes P450, Nuclear Receptors, and Membrane Transporters in 10- and 25-Week-Old db/db Mice", vol. 38, no. 12, 17 December 2010 (2010-12-17), US, pages 2252 - 2258, XP055853029, ISSN: 0090-9556, Retrieved from the Internet <URL:http://dx.doi.org/10.1124/dmd.110.034223> DOI: 10.1124/dmd.110.034223**

EP 3 775 260 B1

- **BRAHIM ACHOUR ET AL: "Liquid Biopsy Enables Quantification of the Abundance and Interindividual Variability of Hepatic Enzymes and Transporters", CLINICAL PHARMACOLOGY AND THERAPEUTICS, vol. 109, no. 1, 3 November 2020 (2020-11-03), US, pages 222 - 232, XP055768106, ISSN: 0009-9236, DOI: 10.1002/cpt.2102**
- **GHARBI ET AL.: "Identification of Reliable Reference Genes for Quantification of MicroRNAs in Serum Samples of Sulfur Mustard-Exposed Veterans", CELL JOURNAL(YAKHTEH), vol. 17, no. 3, 2015, pages 494 - 501, XP055639078**
- **KORUK ET AL.: "Serum levels of acute phase proteins in patients with nonalcoholic steatohepatitis", TURK J GASTROENTEROL, vol. 14, no. 1, 2003, pages 12 - 17, XP055639103**

Remarks:

The complete document including Reference Table(s) and the Sequence Listing(s) can be downloaded from the EPO website

**Description**

REFERENCE TO SEQUENCE LISTING, TABLE OR COMPUTER PROGRAM

The Sequence Listing is concurrently submitted herewith with the specification as an ASCII formatted text file via EFS-Web with a file name of Sequence Listing.txt with a creation date of March 27, 2019, and a size of 1.3 kilobytes. The Sequence Listing filed via EFS-Web is part of the specification and is hereby incorporated in its entirety by reference herein.

FIELD OF THE INVENTION

The present disclosure is directed towards simulation systems, methods and apparatus for the predictive modelling of clearance and metabolism of drugs and other substances within individual animals, such as humans. The invention is directed to the corresponding method as defined by the claims.

BACKGROUND OF THE INVENTION

Differences in drug absorption, metabolism and clearance in any given individual may depend on a plurality of causes such as age, gender, and ethnicity to name only a few. In addition, lifestyle choices and health status can have profound effects upon a given individual's ability to absorb, distribute, metabolise or excrete pharmaceutical and other xenobiotic substances. For example, whether an individual is a smoker or suffers from a chronic disease, such as cirrhosis, will influence his/her body's ability to clear the drug. The observation of extreme side-effects or the unexpected lack of expected therapeutic effects in some individuals following administration of putatively therapeutic doses of a drug, thus, requires a fuller understanding of the issue of variability and highlights the importance of identifying covariates that determine the exposure to drugs in each individual. Although genetics may determine some variations in biological activities of organs on a drug (e.g. the genotype of an enzyme) and also of drugs on the body (pharmacodynamics), within any given genotype there are still variations which cannot be predicted with genotyping as it is carried out currently.

There exists a need to provide a more accurate way to identify safe and efficacious drug dosage for individuals in need rather than current crude population-based measures. The various factors that impact pharmacokinetics and pharmacodynamics in paediatric and geriatric contexts, for example, are quite different to those of adults (Zhou et al. Clin Pharmacol Ther. (2018) Jul;104(1):188-200). Even between adults there are considerable variations between population cohorts as a result of *inter alia* obesity, liver and/or renal impairment (Spanakis & Marias, (2014) In Silico Pharmacol. Dec;2(1):2). As a consequence, there is often a need to continually monitor and modify drug dose regimens in the elderly; neonates, infants, and children; as well in various adult sub-populations. Variations with a similar level of profound effect may also exist in other sub-populations and yet may be effectively disregarded by companies or clinicians when drugs are developed or prescribed.

Physiologically-based pharmacokinetic (PBPK) modelling in combination with classical population pharmacokinetic (popPK) model-based simulations is used increasingly to answer questions in drug development in order to modify dosing and dosage regimen of drugs. It is, of course, possible to determine kinetics and dynamics for a particular drug or xenobiotic in an individual and accordingly change the dose. Such an approach requires building a model for each given drug/xenobiotic in each individual as opposed to creating a virtual mirror image (Virtual Twin) of the individual which can handle *any* given drug or xenobiotic in the same way that the individual themselves will handle it. The construction of Virtual Twin PDPK models has been described in US Patent Application No 2016/0335412.

Building an accurate model for each individual (Virtual Twin), *a priori* to administration of any drug or exposure to any xenobiotic compound, requires biopsies to be taken of the most relevant tissue(s), particularly those responsible for clearance of the particular compound under study, which is often the liver. It is evident that it is impractical and potentially hazardous to expect individuals to be subjected to invasive biopsy procedures to harvest tissue from, say, the liver and kidney, simply to create an individualised model of drug metabolism and clearance. In addition, it is difficult and ethically challenging to explore complex clinical scenarios such as drug-drug interactions (DDIs) in paediatric contexts or in small sub-populations having rare genetic variations of drug metabolizing enzymes. As a result of such complexity and inconvenience there has been little effort in the field to pursue models that can provide accurate predictions of drug absorption, distribution, clearance and metabolism at the individual level. Hence, there exists a barrier to the creation of PBPK models that allow for the adoption of personalised point of care dosage regimens for drugs. Consequently, the problem of over- and under-dosing, as well as failure to predict adverse DDIs, is perpetuated.

Cell free nucleic acids are present in the bloodstream and include RNA, so-called 'circulating RNA', despite the typically very short half-life of RNA outside cells (El-Hefnawy et al., Clin Chem, 2004). RNA molecules of this nature therefore are indicated to be associated with lipids, such as vesicles and lipoproteins, to enable their survival. Circulating RNA includes mRNA, which can be enriched in microvesicles or exosomes released by cells.

WO-A-02/00935 (Ramanathan) provides a description of a method for estimating the levels of certain drug metabolizing enzymes in liver - so-called "drug clearance markers" - by correlating to levels of mRNAs found in the blood cells of an individual. In Ramanathan, mRNA is isolated from a blood sample, and reverse transcribed to form cDNA, which is then analysed on a DNA microarray in order to estimate the presence and amount of protein levels of drug clearance markers in the liver based on the corresponding levels of hepatic mRNA expression. There are problems in the methodology of Ramanathan because it relies upon two assumptions:

1) that there is a direct correlation between the levels of mRNA in the blood with corresponding levels of mRNA for a given enzyme or transporter in the liver of that individual; and
2) that the individual liver mRNA levels correspond in a linear fashion to the amount of protein of the same liver enzymes and transporter present in that individual.

Ramanathan's own experiments rely on correlations between tested levels of mRNAs of different enzymes in blood samples from a first group of individuals with previously reported prior art measures of corresponding liver enzymes from a second group of different individuals. Hence, no meaningful correlations can be determined from the Ramanathan studies for any given specific enzyme and transporter as the alleged correlation occurs for a set of different enzymes and transporters between samples taken from blood and liver of different individuals. Indeed, many factors affect the translation of mRNAs into a given protein and that for any one given gene expression product there may be multiple regulatory mechanisms that control its translation into a corresponding functional protein. In the case of human-derived hepatocytes, for a several thousandfold increase in mRNA, there might be only a few-fold change in the actual level of protein (as reviewed by Einolf et al, Clin Pharmacol Ther. 2014 Feb;95(2):179-88). Such effects may also be highly susceptible to environmental, genetic and lifestyle factors that can modulate the level and activities of drug clearance enzymes *in vivo* on an individual basis.

A further complication arises from a phenomenon described as "shedding" of mRNA by the cells of an organ or tissue often within exosomes into bodily fluids, such as into the bloodstream. The amount of shedding varies between individuals with "fast shedders" releasing a higher amount of RNA for the same amount of transcription of a particular gene in the originating organ or tissue when compared to that released by "slow shedders". It can be appreciated, therefore, that quantification of circulating RNA alone without correction for the level of shedding within an individual will be only of limited use in accurately predicting the protein levels derived from expression of a particular gene in organ tissue. Hence, assertions in the art that circulating mRNA, of exosomal or other origin, may serve as a source of "liquid biopsy" for correlation with abundance of organ drug handling proteins are at best speculative and at worst highly premature in addressing the significant technical problems that exist. Van der Hauwaert et al (Toxicology and Applied Pharmacology; Vol 279: 3, September 2014) discusses a study evaluating the mRNA expression level of 377 genes encoding xenobiotic-metabolizing enzymes (XMEs), transporters, as well as nuclear receptors and transcription factors that coordinate their expression in eight normal human renal cortical tissues. Lam et al (Drug Metabolism and Disposition; Volume 38, Issue 12, December 2010) discusses a study characterizing changes in the expression and in some cases the function of a diverse set of genes involved in drug metabolism, transport, and regulation in a commonly used diabetic db/db mouse model.

Hence, there remains a need to provide a practical means for generating an accurate and quantitative profile of the levels of each protein relevant to drug and other xenobiotic handling in the body of an individual. Such a profile can provide personalised dosing information in conjunction with models defining the Virtual Twin of an individual, and increase the likelihood of determining desired response (or avoidance of reactions) to administration of a specified drug (or exposure to a toxin or environmental chemical or other xenobiotic compound).

These and other uses, features and advantages of the invention should be apparent to those skilled in the art from the teachings provided herein,

## SUMMARY OF THE INVENTION

To answer the limitations of previous approaches the present inventors have recognized that levels of mRNA in a liquid biopsy, such as blood, and in the corresponding organ, such as liver, need to be normalized and individualized using a factor described as a "Shedding Correction Factor (SCF)".

Accordingly, a first aspect of the invention provides a method for identifying clearance and/or metabolic capacity of an individual subject for a specified xenobiotic compound, the method comprising:

(1) identifying at least one protein responsible for the clearance of the xenobiotic compound in a human or animal;
(2) quantifying an amount of a first cell free RNA (cfRNA) present in a liquid biopsy obtained from the individual subject, wherein the first cfRNA is derived from an organ within the body of the subject and wherein the first cfRNA codes for the at least one protein responsible for the clearance of the xenobiotic compound, the quantification comprising:

isolating total cell free RNA (cfRNATOTAL) from the liquid biopsy;

analysing the isolated cfRNATOTAL in order to determine an amount of the first cfRNA present within the cfRNATOTAL; and

performing a normalizing function on the amount of the first cfRNA present against an RNA organ Shedding Correction Factor (SCF) that is determined for the subject by;

(i) performing an analysis of the cfRNATOTAL in order to quantify an amount of mRNA present within the cfRNATOTAL that corresponds to each of two or more marker genes, wherein a marker gene is defined as a gene that is expressed principally and consistently in the organ; and

(ii) determining SCF as the mean concentration of mRNA of the each of two or more marker genes present within the cfRNATOTAL.

(3) identifying the abundance of the at least one protein responsible for the clearance of the xenobiotic compound within an organ of the subject by comparison of the amount of first cfRNA encoding the at least one protein responsible for the clearance of the xenobiotic compound with an abundance curve generated by comparison of matched samples comprising cfRNA levels from liquid biopsy and levels of the protein responsible for the clearance of the xenobiotic compound from tissue biopsy from one or more reference individuals, wherein the matched samples are obtained from the same reference individual; and

(4) identifying the clearance capacity of the individual subject based upon the abundance of the protein responsible for the clearance of the xenobiotic compound within the organ of the subject.

In one embodiment of the invention, the SCF is determined for the subject by isolating $cfRNA_{TOTAL}$ from a liquid biopsy obtained from the subject, performing an analysis of the $cfRNA_{TOTAL}$ in order to quantify an amount of two or more marker genes mRNAs present, designated as $[cfRNA]_{Marker}$, wherein a marker gene is defined as a gene that is expressed principally and consistently in the organ and at a high level; and determining the SCF according to the formula A:

$$SCF = \sum_{i=1}^{N}[cfRNA]_{Marker_i}/(N \times [cfRNA]_{TOTAL}) \qquad \textbf{A}$$

where N is equal to the number of marker genes quantified. Optionally at least three marker genes are selected in order to determine the SCF.

Suitably, at least three, suitably at least five, typically at least eight and optionally at least ten or more marker genes are selected in order to determine the SCF. Optionally, the organ is selected from one or more of the group consisting of: the liver; the kidney; the gut; the brain; and the pancreas. In a specific embodiment the organ is the liver.

Where the organ is the liver, then at least one of the two or more marker genes may be selected from the group consisting of: A1BG (Alpha-1-B glycoprotein); AHSG (alpha-2-HS-glycoprotein); ALB (Albumin); APOA2 (Apolipoprotein A-II); C9 (Complement component 9); CFHR2 (Complement factor H-related 5); F2 (Coagulation factor II (thrombin)); F9 (Coagulation factor IX); HPX (Hemopexin); SPP2 (Secreted phosphoprotein 2); TF (Transferrin); MBL2 (mannose-binding lectin (protein C) 2); SERPINC1 (Serpin peptidase inhibitor, clade C (antithrombin), member 1); and FGB (Fibrinogen beta chain).

Typically, the first cfRNA encodes an organ protein. Suitably, the organ-derived cfRNA encodes a xenobiotic handling protein selected from the group consisting of: a xenobiotic clearance protein; a xenobiotic metabolising enzyme; and a xenobiotic transporting protein. In embodiments of the invention, the xenobiotic is a pharmaceutical compound or drug. Optionally, the first cfRNA encodes an enzyme. In one embodiment of the invention, the enzyme comprises a cytochrome P450 monooxygenase (CYP) protein. Suitably, the CYP is selected from one of the group consisting of: CYP1A1; CYP1A2; CYP1B1; CYP2A6; CYP2A7; CYP2A13; CYP2B6; CYP2C8; CYP2C9; CYP2C18; CYP2C19; CYP2D6; CYP2E1; CYP3A4; CYP3A5; and CYP3A7. In a further embodiment, the enzyme comprises a transferase selected from one of the group consisting of: a methyltransferase; a sulfotransferase; an N-acetyltransferase; a glucuronosyltransferase including, but not limited to, one or more of the group consisting of UGT1A1, UGT1A3, UGT1A4, UGT1A6, UGT1A9, UGT2B4, UGT2B7, and UGT2B15; a glutathione-S-transferase; and a choline acetyl transferase.

In another embodiment, the transport protein is an ATP-binding cassette (ABC) transporter or a solute carrier (SLC) transporter.

According to the present disclosure, the liquid biopsy comprises a sample of a bodily fluid selected from one of the group consisting of: blood; urine; saliva; semen; tears; lymphatic fluid; stool; and a mucus secretion. In embodiments where the liquid biopsy comprises blood or a component thereof, it may comprise whole blood, serum and/or plasma.

According to the present disclosure, the method further provides for quantifying the amount of at least a second cell free RNA (cfRNA), or a third, fourth, fifth or more cfRNA present in the liquid biopsy. In a specific embodiment, a plurality of cfRNAs are quantified each one of the plurality of cfRNAs corresponding to a different organ protein as defined herein.

According to the present disclosure, is provided an *in vitro* assay for quantifying the amount of at least a first cell free RNA (cfRNA) present in a blood sample that has been obtained from an individual subject, wherein the first cfRNA encodes a drug clearance or metabolising protein derived from the liver of the subject, and wherein the first cfRNA is comprised within an microsome present in the blood sample, the assay comprising apparatus configured to perform the steps of:

> a. isolating total cell free RNA (cfRNA$_{TOTAL}$) from the blood sample;
> b. analysing the isolated cfRNA$_{TOTAL}$ in order to determine a concentration of the first cfRNA present within the cfRNA$_{TOTAL}$; and
> c. normalizing the concentration of the first cfRNA present against a RNA liver Shedding Correction Factor (SCF) that is determined for the subject, wherein the SCF is defined by performing an analysis of the cfRNA$_{TOTAL}$ in order to quantify an amount of mRNA present within the cfRNA$_{TOTAL}$ that corresponds to each of a plurality of liver marker genes; and determining the SCF as the mean concentration of mRNA of the each of plurality of liver marker genes present within the cfRNA$_{TOTAL}$.

Accordingly, the SCF is determined for the subject by isolating cfRNA$_{TOTAL}$ from a blood sample obtained from the subject, performing an analysis of the cfRNA$_{TOTAL}$ in order to quantify an amount of liver marker genes mRNAs present, designated as [cfRNA]$_{Marker}$, wherein a liver marker gene is defined as a gene that is expressed principally and consistently in the liver and at a high level; and determining the SCF according to the formula A:

$$SCF = \sum_{i=1}^{N}[cfRNA]_{Marker_i}/(N \times [cfRNA]_{TOTAL}) \qquad \mathbf{A}$$

where N is equal to the number of liver marker genes quantified.

Suitably, at least one of the liver marker genes is selected from the group consisting of: A1BG (Alpha-1-B glycoprotein); AHSG (alpha-2-HS-glycoprotein); ALB (Albumin); APOA2 (Apolipoprotein A-II); C9 (Complement component 9); CFHR2 (Complement factor H-related 5); F2 (Coagulation factor II (thrombin)); F9 (Coagulation factor IX); HPX (Hemopexin); SPP2 (Secreted phosphoprotein 2); TF (Transferrin); MBL2 (mannose-binding lectin (protein C) 2); SERPINC1 (Serpin peptidase inhibitor, clade C (antithrombin), member 1); and FGB (Fibrinogen beta chain).

According to the present disclosure is provided a method for identifying clearance and/or metabolic capacity of an individual subject for a specified xenobiotic compound, the method comprising:

> (1) identifying at least one xenobiotic clearance protein that contributes to pharmacokinetics of the specified xenobiotic compound in a human or animal;
> (2) quantifying an amount of cell free RNA encoding the at least one xenobiotic clearance protein within a liquid biopsy obtained from the subject, according to the methods as described herein; and
> (3) identifying the abundance of the at least one xenobiotic clearance protein within an organ of the subject by comparison of the amount of cell free RNA encoding the at least one xenobiotic clearance protein with an abundance curve for the corresponding amount of xenobiotic clearance protein in the organ; and
> (4) identifying the clearance capacity of the individual subject based upon the abundance of the at least one xenobiotic clearance protein within the organ of the subject.

Suitably, the organ is selected from the group consisting of: the liver; the kidney; the gut; the brain; and the pancreas. Typically, the organ is the liver.

According to the present disclosure the abundance curve is generated by comparison of matched samples comprising a liquid biopsy with a tissue/organ biopsy from a reference individual. Suitably, the matched samples are obtained from the same individual.

According to the present disclosure is provided a system for modelling clearance and/or metabolic capacity of an individual subject for a specified xenobiotic compound, the system comprising:

> an input device, for inputting data relating to the subject;
> a computer readable medium containing program instructions for implementing a method as described herein, wherein execution of the program instructions results in one or more processors of the system carrying out the steps of the method; and

an output device for presenting a model of clearance capacity for the specified xenobiotic compound for the individual.

According to the present disclosure,, the input device and the output device are the same device.

Optionally, the input device and the output device comprise a user interface device.

According to the present disclosure,the computer readable medium is located with a first server.

According to the present disclosure, the first server is located remotely from the input device. According to the present disclosure, the first server is located remotely from the output device. Optionally, the first server is configured to communicate with at least a second or further servers. Suitably, the at least a second (or further) server provides additional modelling capability, including at least one physiologically-based pharmacokinetic (PBPK) model.

According to the present disclosure is provideda computer server comprising:

a computer readable medium containing program instructions for implementing a method as described herein, wherein execution of the program instructions results in one or more processors of the server carrying out the steps of the method and producing an *in silico* model of clearance capacity of an individual subject for a specified xenobiotic compound, wherein the model is hosted on the server; and
a telecommunication module for communicating with a remotely located user interface device, thereby permitting a remotely located user to access the model.

According to the present disclosure,, the server is located remotely from the user interface.

According to the present disclosure is provided a method of treating a subject in need thereof with a specified pharmaceutical or therapeutic composition, the method comprising:

determining a personalised clearance capacity for the subject for the pharmaceutical or therapeutic composition by a method as described herein; and
generating an optimised dosage regimen for the subject based upon the determination of personalised clearance capacity.

It will be appreciated that the features of the invention may be subjected to further combinations not explicitly recited above, provided that they still fall within the scope of the claims.

DRAWINGS

The disclosure is further illustrated by reference to the accompanying drawings in which:

Figure 1A shows an illustration of mRNA shedding from an organ, in this case the liver.

Figure 1B shows the level and inter-individual variability in expression of selected marker genes from liver as measured by circulating RNA, used to define a shedding correction factor, including data from healthy individuals and cancer patients.

Figure 2A shows the measured plasma expression levels of four enzymes in cancer patients corrected for shedding using a shedding correction factor and the quantified protein abundance levels of these enzymes in matched organ tissue (liver).

Figure 2B shows correlation between the measured plasma expression levels of four enzymes in cancer patients, corrected for shedding, and the quantified protein abundance levels of these enzymes in matched organ tissue (liver).

Figure 3 shows weak correlation between the measured plasma expression levels of four enzymes in cancer patients, without correction for shedding, and the quantified protein abundance levels of these enzymes in matched liver tissue.

Figure 4 shows a schematic of an embodiment of a system of the disclosure that creates an *in silico* virtual twin model of an individual subject based upon data obtained from a liquid biopsy from the subject in combination with computer based simulation models.

Figure 5 shows a schematic of an embodiment of a system of the disclosure that creates an *in silico* virtual twin model of an individual subject based upon data obtained from a liquid biopsy from the subject in combination with computer based simulation models. The data is then used to establish a personalised dosage regimen for a therapeutic treatment to be administered to the subject.

DETAILED DESCRIPTION OF THE INVENTION

The invention is defined by the claims.

Hereinafter "embodiments" are embodiments of the invention only if they fall within the scope of the claims. Otherwise they are mere embodiments of the disclosure.

Unless otherwise indicated, the practice of the present invention employs techniques of chemistry, computer science, statistics, molecular biology, microbiology, recombinant DNA technology, and chemical methods, which are within the comprehension of a person of ordinary skill in the art. Such techniques are also explained in the literature, for example, T. Cormen, C. Leiserson, R. Rivest, 2009, Introduction to Algorithms, 3rd Edition, The MIT Press, Cambridge, MA; L. Eriksson, E. Johansson, N. Kettaneh-Wold, J. Trygg, C. Wikstom, S. Wold, Multi- and Megavariate Data Analysis, Part 1, 2nd Edition, 2006, UMetrics, UMetrics AB, Sweden; M.R. Green, J. Sambrook, 2012, Molecular Cloning: A Laboratory Manual, Fourth Edition, Books 1-3, Cold Spring Harbor Laboratory Press, Cold Spring Harbor, NY; Ausubel, F. M. et al. (1995 and periodic supplements; Current Protocols in Molecular Biology, ch. 9, 13, and 16, John Wiley & Sons, New York, N. Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridisation: Principles and Practice, Oxford University Press; M. J. Gait (Editor), 1984, Oligonucleotide Synthesis: A Practical Approach, IRL Press; and D. M. J. Lilley and J. E. Dahlberg, 1992, Methods of Enzymology: DNA Structure Part A: Synthesis and Physical Analysis of DNA Methods in Enzymology, Academic Press.

An embodiment of the present invention includes a modelling and simulation based algorithm, together with associated hardware, which integrates *in silico*, *in vitro*, and *in vivo* preclinical data from a wide range of sources with mechanism-based models to anticipate and predict the exposure and effects of drugs in humans or animals. The algorithm utilizes empirical and descriptive models to describe the linkage between drug - or other xenobiotic - concentration, and observed response in various body tissues on drug clearance, especially in organs such as the liver, kidney, brain or gut. In addition, the methods and apparatus utilise absorption, distribution, metabolism and excretion databases for 'bottom-up' mechanistic modelling and simulation of the processes of oral absorption, tissue distribution, metabolism and excretion of drugs and drug candidates in healthy and diseased individuals.

Prior to setting forth the invention, a number of definitions are provided that will assist in the understanding of the invention. Unless otherwise defined, all technical and scientific terms used herein have the same meaning as commonly understood by one of ordinary skill in the art to which this invention belongs.

As used herein, the term "comprising" means any of the recited elements are necessarily included and other elements may optionally be included as well. "Consisting essentially of" means any recited elements are necessarily included, elements that would materially affect the basic and novel characteristics of the listed elements are excluded, and other elements may optionally be included. "Consisting of" means that all elements other than those listed are excluded. Embodiments defined by each of these terms are within the scope of this invention.

The term "nucleic acid" as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acids may include DNA and RNA, subtypes of these such as genomic DNA, mRNA, miRNA, tRNA and rRNA, and may be manufactured synthetically in vitro or isolated from natural sources. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

The term "amino acid" in the context of the present invention is used in its broadest sense and is meant to include naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term "amino acid" further includes D-amino acids, retro-inverso amino acids as well as chemically modified amino acids such as amino acid analogues, naturally occurring amino acids that are not usually incorporated into proteins such as norleucine, and chemically synthesised compounds having properties that are characteristic of an amino acid, such as β-amino acids. For example, analogues or mimetics of phenylalanine or proline, which allow the same conformational restriction of the peptide compounds as do natural Phe or Pro, are included within the definition of amino acid. Such analogues and mimetics are referred to herein as "functional equivalents" of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983.

A "polypeptide" is a polymer of amino acid residues joined by peptide bonds, whether produced naturally or in vitro by synthetic means. Polypeptides of less than around 12 amino acid residues in length are typically referred to as "peptides" and those between about 12 and about 30 amino acid residues in length may be referred to as "oligopeptides". The term "polypeptide" as used herein denotes the product of a naturally occurring polypeptide, precursor form or proprotein. Polypeptides can also undergo maturation or post-translational modification processes that may include, but are not limited to: glycosylation, proteolytic cleavage, lipidization, signal peptide cleavage, propeptide cleavage, phosphorylation, and such like. The term "protein" is used herein to refer to a macromolecule comprising one or more polypeptide chains.

As used herein the term "biomarkers" may comprise cells, cellular components, peptides, polypeptides proteins, ncRNA, genomic DNA, metabolites, cytokines, antigens, and polysaccharides; as well as physiological parameters such as cell count, temperature, $O_2$ level, $CO_2$ level, or pH. Biomarkers may also comprise mRNA coding for polypeptides that are not involved in xenobiotic clearance. Suitably, the biomarkers comprise a combination of features.

The term "levels" is used herein to define terms of quantity or abundance of a specified factor and may be defined in molar or absolute amounts (i.e. micrograms or milligrams etc.), concentration (e.g. mg ml$^{-1}$ or mol g$^{-1}$ etc.), and/or in terms of a specific activity (e.g. units of activity in a standard assay). The selected "level" will be appreciated as appropriate to a given factor, for example, where it is appropriate to define the amount of a given enzymatic factor by its specific activity, it may be that this measure is selected rather than the actual amount (in mg/ml) of that factor that may be present. The term "normal level", when in the context of levels of gene or polypeptide expression, is used herein to denote the level of gene expression or enzymic activity in healthy non-diseased samples. Normal levels of expression or activity represent the baseline or control level of expression of a gene. Aberrant levels in cells, either at levels that are too high or too low, are considered not to be normal and can be indicative of disease in the samples from which the cells have been obtained, e.g. cancer.

The term "allelic variant" is used herein to denote any two or more alternative forms of a gene occupying the same chromosomal locus and controlling the same inherited characteristic. Allelic variation arises naturally though mutation, and may result in phenotypic polymorphism within populations. Gene mutations typically result in an altered nucleic acid sequence and in some cases an altered polypeptide sequence also. As used herein, the term "allelic variant" is additionally used to refer to the protein or polypeptide encoded by the allelic variant of a gene.

An "antibody" denotes a protein that is produced in response to an antigen that is able to combine with and bind to the antigen, preferably at a specific site on the antigen, known as an epitope. The term as used herein includes antibodies of polyclonal and monoclonal origin, unless stated otherwise. Polyclonal antibodies are a group of antibodies produced by different B lymphocytes in response to the same antigen; different antibodies in the group typically recognize different parts (epitopes) on the antigen. A monoclonal antibody recognizes only one type of antigen and is produced by the daughter cells of a single antibody-producing lymphocyte, typically a hybridoma. Also included within the term 'antibody' are fragments, such as the Fab, F(ab')2 and Fc portions, as well as derivatives of antibodies, such as chimeric fusions with labelling moieties including green fluorescent protein (GFP).

An "antigen" denotes a molecule that triggers an immune response. An antigen may be in the form of a full length polypeptide or protein. Alternatively, the antigen can be in the form of peptide fragments that bear the specific epitopes that allow antibodies raised against such fragments to also bind to the full length polypeptide.

The term "isolated", when applied to a polynucleotide sequence, denotes that the sequence has been removed from its natural organism of origin and is, thus, free of extraneous or unwanted coding or regulatory sequences. The isolated sequence is suitable for use in recombinant DNA processes and within genetically engineered protein synthesis systems. Such isolated sequences include cDNAs and genomic clones. The isolated sequences may be limited to a protein encoding sequence only, or can also include 5' and 3' regulatory sequences such as promoters and transcriptional terminators.

The term "isolated", when applied to a polypeptide is a polypeptide that has been removed from its natural organism of origin. It is preferred that the isolated polypeptide is substantially free of other polypeptides native to the proteome of the originating organism. It is most preferred that the isolated polypeptide be in a form that is at least 95% pure, more preferably greater than 99% pure. In the present context, the term "isolated" is intended to include the same polypeptide in alternative physical forms whether it is in the native form, denatured form, dimeric/multimeric, glycosylated, crystallised, or in derivatized forms.

As used herein, the term "organ" is synonymous with an "organ system" and refers to a combination of tissues and/or cell types that may be compartmentalised within the body of a subject to provide a biological function, such as a physiological, anatomical, homeostatic or endocrine function. Suitably, organs or organ systems may mean a vascularized internal organ, such as a liver, kidney, brain or pancreas; or may comprise fluid organ systems such as the blood and circulatory system. Typically organs comprise at least two tissue types, and/or a plurality of cell types that exhibit a phenotype characteristic of the organ.

The term "sample" is used to describe isolated materials of biological origin that can be used for a diagnostic, analytical or prognostic purpose. Biological materials may be analysed in tissue microarrays, or via other assay methods, and can include tissues from specific organs such as liver, kidney, brain, heart, epithelium, lung, and bone, as well as other tissues; as well as fluid materials such as whole blood, plasma, serum, lymph, urine, stool, cerebrospinal fluid and saliva etc. Such materials may also include in vivo and in vitro cellular materials such as healthy or diseased cells and cell lines - e.g. cancer cell lines, which may be manipulated for in vitro purposes - e.g. immortalised cell lines or induced pluripotent stem cells. The macromolecules analysed in these materials typically include polypeptides such as proteins as well as polynucleotides such as RNA (including mRNA), and DNA. The term "blood sample" may refer to any or all of whole blood, plasma, serum, erythrocyte and/or leucocyte fractions, and any other blood derivative.

The term "microsome" refers to vesicles made by re-forming of the endoplasmic reticulum (ER) during the break-up of cells *in vitro,* which can be concentrated and isolated from other cell debris. Cytochrome P450 monooxygenase enzymes (CYPs) are present in ER and so microsomal preparations containing CYPs can be obtained from tissue samples such as organ tissue (e.g. liver), where CYPs are highly abundant. CYPs are further discussed below.

The term "microvesicle" or "exosome" relates to extracellular vesicles that may be produced or shed by cells for example by exocytosis, budding or blebbing of the plasma membrane. Cell death by apoptosis may also lead to microvesicle production. Microvesicles are found in interstitial space and in many body fluids, and may contain mRNA, miRNA and/or proteins. It is thought that methods of intercellular communication may rely on microvesicle transport. Exosomes are a type of microvesicle.

"Cell free nucleic acid" may be DNA, RNA, or any combination thereof. The nucleic acid may be cell free DNA (cfDNA), cell free RNA (cfRNA), or any combination thereof. The samples from which the cell free nucleic acids may be isolated include any bodily fluid capable of providing a liquid biopsy. Where the liquid biopsy comprises blood, the cell free nucleic acids may be located within plasma or serum.

As used herein, the phrases "drug metabolizing enzymes" or "drug clearance proteins" will include cytochrome P450 monooxygenase enzymes (CYPs) as well as membrane transport proteins, and transferases. In embodiments of the invention the CYP enzymes are selected from human CYP families 1, 2 and 3, which are the CYP families typically linked to xenobiotic (e.g. drug) metabolism and clearance. Suitably the CYPs may comprise any, some or all of the CYPs selected from the group consisting of: CYP1A1; CYP1A2; CYP1B1; CYP2A6; CYP2A7, CYP2A13; CYP2B6; CYP2C8; CYP2C9; CYP2C18; CYP2C19; CYP2D6; CYP2E1; CYP3A4; CYP3A5 and CYP3A7. CYPs are haemoproteins, that is, of the superfamily of proteins containing haem (or heme) as a cofactor. These proteins are involved in the metabolism of xenobiotics, in general by oxidation reactions involving NADPH and oxygen. Different drugs often have different CYP proteins involved in their metabolism, a selection of exemplary compounds that are substrates for corresponding metabolizing CYPs are listed below - it will be appreciated that this list is non-exhaustive:-

| | |
|---|---|
| **CYP1A2** | Caffeine; Tacrine; Theophylline; Melatonin; Clozapine; Lidocaine |
| **CY2A6** | Bilirubin; Cortinine; Coumarin |
| **CYP2B6** | Benzphetamine; Buproprion; Methamphetamine; Temazepam; |
| **CYP2C8** | Amodiaquine; Paclitaxel; Ibuprofen |
| **CYP2C9** | Diclofenac; Irbesartan; Valsartan; Ibuprofen; Tamoxifen; Tolbutamide |
| **CYP2C19** | Hexobarbital; Imipramine; Melatonin; Omeprazole; Diazepam |
| **CYP2D6** | Codeine; Dihydrocodeine; Amphetamine; Loratidine; Oxycodone; Paroxetine; Risperidone; Tamoxifen |
| **CYP2E1** | Aniline; Chlorzoxasone; Halothane; Isoflurane; para-Nitrophenol; Vinylchloride |
| **CYP3A4/5** | Alfentanil, Alprazolam; Atorvastin; Cortisol; Cholesterol; Dasatinib; Dexamethasone; Diazepam; Midazolam; Prednisolone; Quinine; Sildenafil; Testosterone; Triazolam; Vincristine |

(Zanger & Schwab (2013) Pharmacology & Therapeutics 138 (2013) 103-141; Watari et al. (2019) Biol. Pharm. Bull. 42, 348-353);

Other, non-CYP, proteins that are involved in metabolism of xenobiotic molecules include transferases: enzymes that catalyse the transfer of a functional group from a donor molecule to a specified substrate molecule (an acceptor) which is typically a drug or other xenobiotic compound. Transferase enzymes involved in drug metabolism are typically those that catalyse conjugation of moieties such as glutathione, methyl groups, acetyl groups, sulfate, and amino acids to a substrate molecule which may include a drug or a metabolite of a drug. Exemplary drug metabolizing transferases may include methyltransferases; sulfotransferases; N-acetyltransferases; glucuronosyltransferases (UDP-glucuronosyltransferases or UGTs) including, but not limited to, one or more of the group consisting of UGT1A1, UGT1A3, UGT1A4, UGT1A6, UGT1A9, UGT2B4, UGT2B7, UGT2B15 and UGT2B17; glutathione-S-transferases; and choline acetyl transferases.

In addition to the above, membrane bound and non-membrane bound transport proteins also may influence the levels of xenobiotic compound uptake and, hence, the levels of metabolism and clearance of a given compound within the body of an individual. Transport proteins may include one or more of the group selected from: transmembrane pumps, transporter proteins, escort proteins, acid transport proteins, cation transport proteins, vesicular transport proteins and anion transport proteins. Exemplary transporter proteins include ATP-binding cassette (ABC) transporters including, but not limited to, one or more of the group selected from: ABCB1/MDR1, ABCB11/BSEP, ABCC2/MRP2, ABCG2/BCRP. Alternatively, solute carrier (SLC) transporters may include one or more of the group consisting of: SLCO1B1/OATP1B1, SLCO1B3/OATP1B3, SLCO1A2/OATP1A2, SLCO2B1/OATP2B1, SLC22A1/OCT1, SLC22A7/OAT2, and SLC47A1/-MATE1.

As used herein, the phrases "organ marker genes" or "marker genes" refer to genes expressed principally in organs associated with drug/xenobiotic clearance, suitably the liver, consistently and at relatively high levels. By "relatively high levels" it is meant that the expression profile of a given marker gene is expressed, usually constitutively, at readily detectable and quantifiable levels. In the present invention, the amount of these marker genes as measured in circulating RNA may be used as an indicator for the degree of shedding taking place in a certain individual. Thus these data on marker gene level may be used as a 'benchmark' to determine an average baseline of shedding in an individual, showing a basal level of organ gene expression. Such data may be used to reduce variation in correlation of other mRNA sample levels to expression levels of genes in the organ. Suitably, organ marker genes indicative of the liver may be selected from any, some or all of: A1BG (Alpha-1-B glycoprotein), AHSG (alpha-2-HS-glycoprotein), ALB (Albumin), APOA2 (Apolipoprotein A-II), C9 (Complement component 9), CFHR2 (Complement factor H-related 5), F2 (Coagulation factor II (thrombin)), F9 (Coagulation factor IX), HPX (Hemopexin), SPP2 (Secreted phosphoprotein 2), TF (Transferrin), MBL2 (mannose-binding lectin (protein C) 2), SERPINC1 (Serpin peptidase inhibitor, clade C (antithrombin), member 1) and FGB (Fibrinogen beta chain). It will be appreciated that the aforementioned list is not exhaustive and a plurality of alternative organ or tissue specific marker genes may be selected from, for example, the organ-specific or tissue-specific proteomes respectively. Often organ specific marker genes will comprise constitutively expressed genes that show relatively constant levels of expression with low variance over time in tissues having normal pathology - e.g. housekeeping genes. Organ or tissue marker genes may be comprised within a 'panel' that comprises a plurality of such genes. Typically, a panel of organ/tissue marker genes would include not less than eight, suitably not less than ten and optionally not less than twelve genes expressed principally in the specified organ, consistently and at relatively high levels. Such marker genes may be derived from healthy tissues or organs, or they may be derived from diseased tissues/organs. In an embodiment of the invention, the tissue comprises neoplastic tissue, which may be benign or malignant.

As used herein the term "shedding" is used to describe the process of mRNA release by cells from organs or tissues, such as liver hepatocytes, into a bodily fluid, in microvesicles, exosomes, or otherwise as cell free mRNA. The present inventors have identified that mRNA shedding can vary in magnitude between subjects or within the same subject depending on, for example, disease state, and affects the correlation between the levels of a particular RNA detected in the blood, plasma or other sample, and the levels of the same mRNA in the cells and tissue of the organ, such as the liver. The term "RNA shedding" is used as a synonym. A "shedding coefficient", organ "shedding correction factor" or "SCF" refers to a scaling factor for an individual which relates to the amount of shedding by their hepatocytes. A "fast shedder" will shed more RNA for the same amount of gene expression than will a "slow shedder", and thus the SCF for such individuals will differ. It is contemplated that the SCF can be calculated from the quantified levels of the cell free RNA (cfRNA) of one or a plurality of organ marker genes, for example from 1, 2, 3, 4, 5, 6, 7, 8, 9, 10, 11, 12 or more genes. If a subset of N markers are used the SCF can be calculated as follows.

$$SCF = \sum_{i=1}^{N} [cfRNA]_{Marker_i} \Big/ \left(N \times [cfRNA]_{TOTAL}\right)$$

Pharmacokinetics (PK) is the study of what happens to a drug when it is administered to and passes through the various organ and tissue compartments within the body of a subject. Drug absorption, distribution, and elimination are subject to multiple interactions dependent in part upon the biological action of each organ on a drug, partitioning of the drug to these organs and tissue volumes (compartments) and blood flows. The absorption (rate and extent of bioavailability), distribution, metabolism and excretion (ADME), and toxicity profiles of any given pharmaceutical or other xenobiotic compound are key deterministic measures of subsequent pharmacodynamics (action of the drug on body) necessary to achieve efficacy without major safety issues prior to an authorisation for use in medicine.

However, the majority of studies and associated models aggregate across populations leading to predictive models and virtual simulations based upon an average response or response in the average representative of a stratified group. This approach is sometimes referred to as population based pharmacokinetic (popPK) modelling. PopPK methodology relies on mathematical models to describe PK data. Clinicians and drug developers often utilise popPK models in order to help guide decision-making across all phases of drug development. As such, the pharmaceutical industry has become increasingly reliant upon popPK models to generate efficacy and safety data in support of applications for regulatory marketing authorisations (i.e., new drug applications 'NDAs' and biologics license applications 'BLAs').

The present invention is based in part upon the determination of the level of one or more mRNAs coding for drug metabolizing enzymes or drug clearance proteins in a liquid biopsy, such as a biological blood sample, via a quantitative analysis of the sample and establishing a correlation, that provides a predictive assay, from these determined levels to the levels - or abundance/concentration - of xenobiotic (e.g. drug) metabolizing and/or transporting enzymes in the organ, and membrane transporters and transferase enzymes in other tissues of an individual. The abundance relationship may be supplemented by reference to standardized curves or log tables generated by comparison of matched samples comprising a liquid biopsy and a tissue biopsy from one or more reference individuals, generally it is preferred that the matched samples are obtained from the same individual.

Variability of drug response between individuals is an important consideration in clinical medicine. One major determinant of drug response variability is hepatic CYP-mediated drug metabolism due to polymorphism and allelic variation, and difference in expression levels across populations. Other variations may be due to polymorphism or difference in expression levels of other key proteins involved in drug clearance including membrane transporter proteins and transferase enzymes. Hence, the analytical data generated by the inventive methods and apparatus provides a key advantage that enables the construction of significantly improved personalised as well as population based pharmacokinetic computer models. These models may be used in the design of improved clinical trials, incorporated into better dosage regimens, or used to predict and inform personalised medicine choices. The individuals tested or treated according to various instances of the disclosure may be healthy or diseased, and human or animal patients. In veterinary contexts, the drug clearance models may require suitable adaptation, although the underlying principles of the invention are consistent. The term "animal" may include mammals such as cats; dogs; mice; guinea pigs; rabbits; primates; horses; as well as livestock including cattle; pigs; sheep; and goats.

In an embodiment of the invention, the levels of mRNAs - suitably cell free mRNAs - that encode drug metabolizing and transporting proteins, including CYPs, transport proteins and transferases, are measured in a liquid biopsy, suitably a blood sample. The concentration or amount of each mRNA in the blood sample thereby correlates to an amount/concentration/abundance of a drug clearance protein, for example, an enzyme or transporter, in the organ or tissue of the individual from which the mRNA originated. The prediction of amount/concentration/abundance of a drug clearance protein based upon the amount or concentration of the mRNA present in the liquid biopsy can be made by consultation with a calibration curve or log table, for instance.

However, for the relationship between the biomarker concentration in the blood sample and the level of the drug clearance or metabolising protein in the organ tissue to be accurate, the inventors have found that the degree of "shedding" in the individual should be taken into account. For this, it has been found that quantifying the levels of cell free RNA that encodes one or more organ or tissue specific marker genes in the sample can give a subject-specific shedding correction factor (SCF) which can be used to scale the biomarker concentration and correct for how much or little RNA shedding each subject demonstrates, thereby yielding SCF-corrected data (see Examples below). The quantification of organ specific marker genes in the blood sample may typically be carried out by the same methods as used to determine mRNA levels for drug metabolizing enzymes or drug clearance proteins in the same blood sample.

Hence, the RNA profile can be used to calibrate a virtual system to provide a baseline metabolic level for that subject individual. Once configured, the system acts as a virtual twin, *in silico* model, for that individual and can be tested to predict the individual's capacity for drug clearance with one or more xenobiotic compounds. The system can be further refined by the addition of information derived from biomarkers found within the same or a different sample, and/or with other individual-specific physiological and/or epidemiological information, which may be gathered by questionnaire, interview, health professional analysis, measurement with medical diagnostic equipment, or similar. Biomarker levels within a sample may be determined by a range of techniques including macromolecule microarray analysis, mass spectrometry (MS) proteomic profiling, quantitative RT-PCR, ELISA or other antibody-based assays, and chromatographic or spectrophotometric techniques.

RNA levels within the blood sample may be detected by a range of methods, including but not limited to polymerase chain reaction (PCR), reverse transcription polymerase chain reaction (RT-PCR), quantitative real time polymerase chain reaction (Q-PCR), gel electrophoresis, capillary electrophoresis, mass spectrometry, fluorescence detection, ultraviolet spectrometry, DNA hybridization, allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. RNA may be reverse-transcribed by any suitable means to produce cDNA before analysis in any combination with the above. RNA levels can be determined by use of nucleic acid hybridisation arrays or real-time PCR.

DNA arrays are solid supports upon which a collection of gene-specific nucleic acids have been placed at defined locations. In array analysis, a nucleic acid-containing sample is labelled and then allowed to hybridise with the gene-specific targets on the array. Based on the amount of nucleic acid from the sample hybridised to target on the array, information is gained about the specific nucleic acid composition of the sample. Array analysis, according to the present disclosure, involves isolating total RNA from a sample comprising cells or microvesicular material, converting the RNA

EP 3 775 260 B1

samples to labelled cDNA via a reverse transcription step, hybridising the labelled cDNA to identical arrays (such as via either a nylon membrane or glass slide solid support), removing any unhybridised cDNA, detecting and quantitating the hybridised cDNA, and determining the quantitative data (e.g. the levels of biomarkers present) from the various samples.

Real-time or quantitative PCR refers to a method which monitors the replication of a nucleotide sample in real-time during the PCR reaction. As well as the normal components, the reaction mixture contains fluorescent probes which may hybridise to any double-stranded nucleotide sequence or else to a specifically chosen complementary sequence. The signal from the fluorescent probes therefore correlates with the number of the target sequences which have been produced during the reaction and can be used to determine the quantity of the target sequence in the original sample.

Analysis of RNA levels (e.g. quantifying the RNA) within the sample allows for adjustment of the generic settings (standard baseline settings) for the simulation algorithm to correspond to those of the individual subject. In this regard the methods of the invention allow for the determination of the biomarker profile generated for the individual to be correlated with the corresponding levels of drug metabolizing, transporting and clearance proteins within the tissues of said individual, such as in the liver, kidney or gut and on the surface of the target cell. This step comprises quantifying the levels (including concentration/amount/activity) of at least one biomarker within the sample from the individual which is correlated to at least one level of a drug metabolizing, transporting and clearance protein or enzyme within the individual's tissues via a defined correlation function or algorithm. Hence, the model used in practising the invention is able to determine a correlation between first input data in the form of a biomarker profile from the sample and the activity/concentration/amount level of drug metabolizing, transporting and clearance protein or enzyme within the individual's tissues. This enables the production of an augmented profile that comprises baseline biomarker data from the original sample together with the correlated (or predicted) level of drug metabolizing, transporting and clearance protein or enzyme within the individual's tissues. The augmented first input data is used to define the starting points for the simulation model used in practising invention, in terms of baseline levels of drug metabolizing, transporting and clearance protein or enzyme within the individual's tissues, optionally in combination with gene identity and expression data including allelic variation and whether certain genes are either up- or down- regulated compared to average (i.e. mean or median) levels within a given population. The term "down-regulated" as used herein denotes a process resulting in decreased expression of one or more genes and/or the proteins encoded by those genes. "Up-regulated" denotes an increase in gene expression and corresponding protein expression.

Additional factors may have a bearing on drug response. These characteristics may be determined by the measurement of biomarkers in a sample, which can be the same or different sample as the liquid biopsy sample used for determination of the one or more mRNAs coding for drug metabolizing enzymes or drug clearance proteins. For example, allelic variations of CYPs, transporter genes or transferases, or any other relevant gene, may be determined from genomic DNA isolated from a liquid biopsy sample or any of a number of biological samples. This can include information not able to be derived from mRNA sequences, such as intron data, epigenetic information and the presence and activity of genomic regulatory features such as promoters, repressors, and so on.

Non-gene expression parameters which may also be relevant for determining drug response may include parameters which can be determined by measurement of biomarkers in one or more liquid biopsy sample, and/or can include physiological and epidemiological information collected by other means. In some embodiments of any aspects of the invention, one or more non-gene expression parameters may be selected from the group consisting of: ethnicity; genotype; age; age group classification; gender; smoking status; presence of chronic disease, including renal impairment, diabetes (type I or type II) or liver cirrhosis; body mass index (BMI); body adiposity index (BAI) or other equivalent measurements of body fat content; waist circumference measurement; waist-to-hip ratio; hydrostatic weighting; average alcohol consumption; pregnancy; allergy status; blood pressure; total blood lipids (e.g. cholesterol); average resting heartbeat; ECG interval measurements including QT interval, QRS duration, and PR intervals; general medical history; familial medical history; or combinations thereof. Such additional parameters may be used to further refine any model, algorithm, simulation or prediction produced by the invention, improving accuracy.

In some aspects, the present invention provides a method that is used to build a robust computer (in silico) predictive model of drug metabolism, in particular drug distribution and clearance, for a specified individual subject. In this way a computer-based model of drug clearance can be matched to any given individual, following a simple blood test, and thereby provides an accurate personal prediction of an individual's capacity to metabolize and/or clear a given drug, xenobiotic, or combination of drugs or xenobiotics. The so-called Virtual Twin model is incorporated into a computer implemented system that can be utilised by, for example, clinicians, academics, patients and pharmaceutical researchers.

According to an embodiment of the invention the method comprises the steps of obtaining a liquid biopsy sample from an individual. The liquid biopsy may suitably comprise a bodily fluid such as any one or more of: blood, urine, saliva, semen, tears, lymphatic fluid, stool or a mucus secretion. This sample can be obtained via a minimally invasive route, and can include deriving blood components such as plasma, serum or other sample from a whole blood liquid biopsy sample. The sample is analysed quantitatively to determine the levels of one or more, typically a plurality, of mRNAs coding for drug metabolizing enzymes or drug clearance proteins in order to derive a profile of the said individual's circulating mRNA. The sample is also analysed to determine the levels of one or more organ specific marker genes in the sample in order to give a subject-specific organ shedding correction factor (SCF). The SCF is used to provide a baseline for the rate or amount of circulating mRNA shed by the organ and is thus used to correct the profile of the individual's circulating mRNA.

The corrected profile defines first input data, which first input data is then used to calibrate a computer-based model of drug clearance. The calibration step thereby enables the creation of an individual model of drug clearance. This individual model accurately predicts the pharmacokinetics and pharmacodynamics of drug metabolism and clearance, for a specified xenobiotic or pharmaceutical compound or combination of same. Hence, the invention allows providing a robust model that simulates the individual pharmacokinetics for a specified subject.

In some embodiments the method further comprises quantitatively analysing a sample to determine the levels of one or more, typically a plurality, of biomarkers present within the sample in order to derive a profile of the said individual's biomarker(s). The sample may be the same or different to that sample for determining circulating RNA, and as such may further include the steps of obtaining a second biological sample from an individual. The sample may be obtained in any suitable way, but may again be obtained via a minimally invasive route, such as a blood, cheek swab, saliva, stool or urine sample. The profile defines biomarker input data, which biomarker input data is then used to further calibrate the computer-based model of drug clearance.

In some embodiments, physiological and/or epidemiological information to obtain non-gene expression data not derivable from sample biomarkers may be obtained from an individual, in order to derive a physiological and/or epidemiological profile of the said individual. Such information may include ethnicity; age; gender; smoking status; body mass index (BMI); body adiposity index (BAI) or other equivalent measurements of body fat content; waist circumference measurement; waist-to-hip ratio; allergy status; blood pressure; average resting heartbeat; ECG interval measurements including QT interval, QRS duration, and PR intervals; general medical history; familial medical history; or combinations thereof. The profile defines personal input data, which is then used to further calibrate the computer-based model of drug clearance.

The virtual simulator used for the present invention provides a sophisticated platform for the analysis of pharmacokinetic outcomes, drug-drug interactions (so-called DDIs) and tissue-specific responses in a given individual, resulting in a comprehensive physiologically-based pharmacokinetic (PBPK) model. PBPK models can comprise nested compartments that represent different tissue functionalities and cell types within an organ system. When assembled, the levels of hierarchical complexity allow for modeling of molecularly-driven events, such as specific metabolic pathways. The blood flows and partition coefficients that link the compartments - i.e. the organ systems - together mathematically are estimated from animal, *in vitro* data, and clinical data. The parameters and compartments are then optimized to fit the model to existing data.

Hence, the present invention provides a significant advantage over and allows enhancement of prior art modelling systems that are largely based upon population level, animal or *in vitro* based responses. In contrast, according to specific embodiments the present invention allows providing a virtual mimic, also referred to as a "Virtual Twin", for an individual. This Virtual Twin may represent an *in silico* model that is configured so as to represent an entirely personalised PBPK model for a given individual. The model may represent the consolidation of multiple data inputs from a variety of sources, including the physiological and/or epidemiological information described above, the genotype as well as a SCF. This approach facilitates the growth of personalised medicine solutions, improved design of dosage regimens and the identification of potentially harmful side effects before a drug, xenobiotic, or combination of same is administered. In addition the present invention may provide a direct correlation between the levels of circulating RNA present in, for example, the blood with the level (e.g. abundance) of drug metabolizing, transporting and clearance proteins within tissues such as the liver in that individual. Previous approaches have only looked to correlate mRNA and/or biomarker levels with estimates of enzyme activity against a specified probe compound, and as a result have struggled to find utility outside of the very limited probe-enzyme system described.

The virtual simulator may also incorporate an *in vitro* to *in vivo* extrapolation (IVIVE) approach to further inform the model. The IVIVE approach establishes virtual populations by building up mechanistic and physiologically based pharmacokinetic (PBPK) models. These models incorporate identified variabilities in demographic and biological (genetic and environmental) components linked to drug-specific physicochemical properties (for example, aqueous and lipid solubilities) and *in vitro* data on absorption, metabolism and transport. The covariate relationships embedded in such models can be complex and nonlinear and can be difficult to resolve by simple linear covariate analysis. The primary advantage of the IVIVE approach is that it maximizes the value of all *in vitro* information previously generated during drug discovery and preclinical development.

The algorithm used in an embodiment of the invention may include consideration of SCF-corrected input data comprising data pairs or even data clusters. Suitably, data derived from mRNA analysis, such as gene expression data for drug clearance genes, may be categorised further via one or more additional gene and non-gene expression parameters, which may be derived from analysis of biomarkers detected in one or more biological samples. Non-gene expression parameters may include physiological and epidemiological information. In some embodiments of any aspects of the invention, one or more non-gene expression parameters may be selected from the group consisting of: ethnicity; genotype; age; age group classification; gender; smoking status; presence of chronic disease, including renal impairment, diabetes or liver cirrhosis; body mass index (BMI); body adiposity index (BAI) or other equivalent measurements of body fat content; waist circumference measurement; waist-to-hip ratio; hydrostatic weighting; average alcohol consumption; pregnancy; allergy status; blood pressure; total blood lipids (e.g. cholesterol); average resting heartbeat; ECG interval measurements including QT interval, QRS duration, and PR intervals; or combinations thereof.

In a specific embodiment of the invention, the described method can be implemented with the aid of one or more computer systems. According to a further instance, an apparatus comprising one or more memories and one or more processors is provided, wherein the one or more memories and the one or more processors are in electronic communication with each other, the one or more memories tangibly encoding a set of instructions for implementing the *in silico* part of the described methods of the invention. In another embodiment the disclosure provides a computer readable medium containing program instructions for implementing the *in silico* part of the method of the invention, wherein execution of the program instructions by one or more processors of a computer system causes the one or more processors to carry out the steps as described herein. Suitably, the data may be stored in a database, and accessed via a server. Suitably, the server is provided with communication modules to receive and send information, and processing modules to carry out the steps described herein. In some embodiments, the data is provided through a cloud service. In preferred embodiments, the method is accessible as a web service. In some embodiments, users may access the service for recordal or retrieval of scores via a website, in a browser. Networking of computers permits various aspects of the *in silico* part of the invention to be carried out, stored in, and shared amongst one or more computer systems locally and at remote sites. Hence, two or more computer systems may be linked using wired or wireless means and may communicate with one another or with other computer systems directly and/or using a publicly-available networking system such as the Internet.

Suitably, the computer system includes at least: an input device, an output device, a storage medium, and a microprocessor). Possible input devices include a keyboard, a computer mouse, a touch screen, and the like. Output devices computer monitor, a liquid-crystal display (LCD), light emitting diode (LED or OLED) computer monitor, virtual reality (VR) headset and the like. In addition, information can be output to a user, a user interface device (e.g. tablet PC, mobile phone), a computer-readable storage medium, or another local or networked computer. Storage media include various types of memory such as a hard disk, RAM, flash memory, and other magnetic, optical, physical, or electronic memory devices. The microprocessor is a computer microprocessor (e.g. CPU) for performing calculations and directing other functions for performing input, output, calculation, and display of data. In one embodiment of the disclosure, the computer processor may comprise an artificial neural network (ANN). In a further embodiment of the disclosure the computer processor may comprise a machine learning algorithm, suitably a machine learning algorithm that has been trained against an appropriate data set.

The simulation platform for use in practising the *in silico* parts of the invention allows for accurate *in silico* simulation of pharmacodynamic and pharmacokinetic responses by combining two primary classes of data. The first class of data is the corrected first input data in the form of mRNA expression for drug clearance proteins, corrected (such as SCF-corrected) and augmented information (as described above) related to the individual. The second class of data is termed "second input data" and relates to the identity of the drug, compound or substance under test. If drug-drug interactions (DDI) are under consideration then there may be a plurality of second input data. These two types of data may be conveniently stored within XML-based file format that can be viewed and accessed via the system graphical user interface (GUI) as well as other tools such as Microsoft Edge™ (Microsoft Corp., Redmond (WA), USA) or Google Chrome (Google LLC, Mountain View (CA), USA). The schema of these files is designed to allow forward compatibility of files over time such that future release versions and new parameters may be added without disrupting what already exists. This allows files created with a current version of the simulator and to be used with later versions when they are released where any possible missing values are automatically replaced with default values. Files may contain a degree of meta data showing varying information including the software version used to create the file.

The corrected first input data and second input data provides the baseline information for initiating a simulation of drug clearance for a given individual. However, it may also be necessary or desirable to create a workspace that provides contextual information about conditions in which the trial is to be undertaken. The workspace file may also be XML-based; however this time it acts as a container for first and second input data as well as any trial/simulation information and user defined settings. The workspace may also be used as a snapshot of the running condition of any simulation. In other words, to reproduce any simulation exactly, all that is needed is a copy of the workspace taken at the time the simulation was run.

The simulation algorithm of the disclosure handles a multiplicity of pharmacokinetic (PK) model combinations, including:

(1) administration of single (small and large molecules) or multiple chemical moieties,
(2) Different absorption models, namely one-compartment, enhanced Compartmental Absorption and Transit (CAT), and Advanced Dissolution, Absorption and Metabolism (ADAM) models,
(3) Different distribution models such as minimal and full PBPK models with different perfusion- and permeability-limited models, including multi-compartment organ, kidney, blood-brain-barrier, intestinal degradation models and an additional multi-compartment user defined organ/tissue, and

(4) Modelling of a plurality of metabolites.

According to one embodiment of the disclosure, PBPK model algorithms are built using ordinary differential equations (ODE) (for example see Jamei M, Marciniak S, Feng K, Barnett A, Tucker G, Rostami-Hodjegan A, Expert Opin Drug Metab Toxicol. 2009 Feb; 5(2):211-23)

The methods of the invention are particularly useful in contributing to improved construction of PBPK models by providing better understanding over how abundance of drug clearance proteins can vary between individuals. Hence, the disclosure provides, in one embodiment, an improved method for creation of computer based models for the determination of clearance of a given xenobiotic molecule (e.g. drug or biological therapeutic) from individual, or when cumulative data is provided, from a population of individuals. The ease of liquid biopsy, which is far less invasive then solid tissue biopsy sampling, is a major factor in contributing to improved construction of computer models that show utility in drug development and clinical trial design. It also enables new models to be created for use in distinct cohorts such as for neonatal and paediatrics as well as in smaller ethnically distinct populations, or for rare diseases, by way of non-limiting example.

The exposure of an individual to a certain drug can be measured by the area under the concentration time curve (AUC). The AUC after administration through any non-parenteral route (such as an oral dose) is dependent on the proportion of the dose that is absorbed and is subsequently available in the systemic circulation. In the case of oral drug administration (the most common route for drug intake), this involves release of the drug from the formulation, passage through the gut wall and then through the liver. The bioavailability of the drug (F) together with the clearance (CL) and the dose of the drug (D) will determine the overall exposure (AUC) according to the following equation 1 below:

$$AUC = \frac{F \times Dose}{CL}$$

Total clearance (CL) is defined as the volume of blood completely cleared of drug per unit time and encompasses clearance by the liver, the kidneys and biliary excretion (in the absence of reabsorption from the gut). Although exposure to the drug is determined only by the dose, clearance and bioavailability, varying shapes of concentration-time profile can occur for a given exposure when the rate of entry (absorption rate, infusion rate etc.) and rate of elimination are changed. Elimination rate is a function of clearance and distribution characteristics.

Since the majority of drugs currently on the market are lipophilic, metabolism is a major route of elimination from the body. It should be noted that overall metabolic clearance is not usually a simple linear function of the organ but it is also dependent on the delivery of the free drug to the site of metabolism. By way of example: hepatic clearance, distribution and metabolism may be determined by factors such as hepatic blood flow, plasma protein and red blood cell binding, and the effects of influx into or efflux from hepatocytes. *In vivo* intrinsic organ clearance has been extrapolated from *in vitro* models using human liver microsomes/exosomes or human hepatocytes in culture. However, to determine whole organ or even systemic clearance requires the combination of intrinsic clearance rates for multiple drug clearance/metabolising enzymes and transporters in different organs and tissues. For each individual the levels of these enzymes will vary, thus resulting in a different level of clearance for that individual.

An expression that estimates the net intrinsic metabolic clearance in total by the whole liver ($CLu_{H,int}$) from data obtained with recombinantly expressed CYP enzymes is given by equation 2, below:

$$\left[ \sum_{j=1}^{n} \left( \sum_{i=1}^{n} ISEF_{ji} \times \frac{V_{\max i}(rhCYP_i) \times CYP_j \text{ abundance}}{K_{mi}(rhCYP_j)} \right) \right] \times MPPGL \times \text{Liver weight}$$

where there are *i* metabolic pathways for each of *j* CYPs, *rh* indicates recombinantly expressed enzyme, Vmax is the maximum rate of metabolism by an individual CYP, Km is the Michaelis constant, MPPGL is the amount of microsomal protein per gram of liver and ISEF is a scaling factor that compensates for any difference in the activity per unit of enzyme between recombinant systems and hepatic enzymes. This expression indicates that inter-individual variability in hepatic intrinsic clearance can be introduced by incorporating variability in several important parameters. One key parameter influencing this model is the liver abundance (e.g. the level) of each CYP in the individual. Other parameters such as MPPGL and liver weight can be estimated based upon height, weight and age of the individual. However, it is the differences in in metabolism that result from CYP abundance, as well as functional, genetic polymorphisms that can be accommodated by knowing the frequency of different genotypes, and by modifying either the enzyme abundance or the intrinsic enzyme activity. Data on changes in the abundance and/or activity of different drug clearance proteins, such as CYPs, is incorporated into the virtual model for use in the present invention in order to predict hepatic clearance in individuals.

Hence, the virtual *in silico* model for use in practising an *in silico* part of an embodiment of the invention comprises algorithms that are able to incorporate *in vitro* data on drug metabolism/clearance and inter-individual variability that is relevant to drug metabolism/clearance in the tissues of the individual concerned. Optionally, the virtual model may further incorporate allometric scaling models. Allometric scaling methodology attempts to predict mean clearance values in humans from those observed in animal species by scaling for body size. The use of an approach that incorporates IVIVE in addition to allometric scaling has the added advantage of being able to assess the likely individual allelic variability in clearance. For example, some allelic variations of CYP enzymes show decreased catalytic activity compared to wild type and, thus, having knowledge of an individual's specific genotype enables *in vitro* data on kinetics to be used to estimate *in vivo* clearance.

Hence, by incorporating *in vitro* information on enzyme inhibition constants (competitive or non-competitive inhibition) into the virtual model used in practising *in silico* parts of embodiments of the present invention it is possible to predict the extent of metabolic drug-drug interactions (mDDI) *in vivo* for any given individual.

Creating a simulator that provides accurate clearance and/or metabolic capacity values for an individual requires the consideration of multiple parameters - amongst other things: size of organ, genotype of certain enzymes, kidney function etc.. However, of critical importance is the affinity of a given xenobiotic compound (e.g. drug) for the drug clearance proteins present and efficiency of each molecule of protein in handling each molecule of xenobiotic compound. This relationship is described as the $K_{cat}$ and is based on intrinsic clearance of the drug by a given enzyme. By way of example, as described previously, certain drugs are metabolized by particular CYPs, transferases and operate through specific membrane transporters, as well as specific combinations of these drug clearance proteins. The $K_{cat}$ for each CYP or transferase with a given drug is a key determining factor ascertained during clinical trials of any pharmaceutical compound. Hence, if the $K_{cat}$ and/or $CL_{int}$ is a key parameter that is determined in all modern drug development programs, then by estimating the abundance of the relevant drug clearance proteins in an individual's organ, such as the liver, it is possible to predict the clearance of that drug in that individual. The virtual simulator, operates by summing up the various clearances and putting them through the appropriate models of the organ, that consider *inter alia* the limitations of blood flow and availability of free drug concentration in the blood to the organ. Previous attempts at creating such models have typically resulted in low success because they relied upon unmatched samples or estimates of activity based upon literature referenced population averaged levels of abundance.

Accordingly, a dosage regimen is provided, in which parameters related to the administration of a drug comprising a pharmaceutical compound or a biological therapeutic agent to a subject are determined in conjunction with that individual's clearance capacity for the compound or agent. More specifically, a liquid biopsy may be obtained from a subject and $cfRNA_{TOTAL}$ analysis performed. From the $cfRNA_{TOTAL}$ analysis a SCF for the subject is able to be determined. PBPK and popPK clearance models are understood for a wide range of approved pharmaceutical compounds and compound classes. In particular, the specific drug metabolising CYPs, transferases and transporters etc. that are relevant for clearance of most compounds and agents and, therefore, the one or more clearance proteins that constitute $[cfRNA]_{Target}$ for the given drug may be identified. The normalized $[cfRNA]_{Target}$ for the specific clearance protein(s) may be determined from the $cfRNA_{TOTAL}$ thereby enabling the abundance of the specific clearance protein(s) within the relevant organs of a given individual to be ascertained. It will be appreciated that having information of this type for any given individual in relation to a proposed a pharmaceutical compound or a biological therapeutic agent that is to be administered enables a precision dosing regimen to be formulated for that individual for the specific drug.

The individual subject may be a recipient of two or more pharmaceutical compounds or biological therapeutic agents, possibly as a result of a combination therapy or otherwise, in this instance there exists a risk of an adverse DDI. Hence, the individual subject's clearance and/or metabolic capacity for each administered therapeutic agent may be determined as described above and then combined with existing simulation models so as to provide a prediction both of clearance capacity and DDI risk. The risk prediction and clearance capacity are used to inform the determination of a dosage regimen for the individual. It is evident that the ability to elucidate clearance capacity and incorporate this into sophisticated PBPK, PKPD and DDI models enables precision dosing at patient level that will reduce unwanted side effects, over- or under-dosing, improve bioavailability of drug and more optimised drug delivery. The consequent impact on health economics is profound as a result of optimisation of drug consumption and reduction of adverse effects across populations.

Reporting of the output data from the modelling system of the disclosure may be achieved via the GUI or via an output file that may comprise a .csv file or spreadsheet, such as Microsoft Excel™ (Microsoft Corp., Redmond (WA), USA) or Google Sheets (Google LLC., Mountain View (CA), USA). By way of non-limiting example, when the reporting process is implemented through the Excel Automation interface which is based on the Office Object Model. The simulation platform uses this technology to create or connect to an Excel application Component Object Model (COM) object, to manipulate and add worksheets as required. Each worksheet is a bespoke output based on the simulation input selections: each cell is effectively created individually with the selection of font (including size and weight), colour (both foreground and background), alignment of text within the cell, number format (based on the users' machine selection) as well as many other specifications. After the output data has been rendered, graphical representations, such as dashboards, charts, pictograms or graphs may are added if applicable. These may include concentration-time profiles or, for example, pie charts of enzyme contribution which are created based on the output data comprised within the worksheet and formatted individually based on user selections such as number format, dashboard arrangement and also the colour 'skin' chosen before displaying the data.

In an alternative embodiment of the disclosure output data is comprised within a relational database. An advantage of this embodiment is that the simulator algorithm may be comprised as part of an organisational workflow as it can then write directly into a corporate database, for example. This enables formatting and visualisation and data analytics to be customised by the user.

Embodiments of the disclosure may also relate to an apparatus or device for performing a set of operations as defined herein, such as a set of operations that may suitably implement at least one *in silico* part of an embodiment of the present invention. The apparatus may be specially constructed for the required purposes, and/or it may comprise a general-purpose computing device selectively activated or reconfigured by a computer program stored in the computer. Such a computer program may be stored in a non-transitory, tangible computer readable storage medium, or any type of medium suitable for storing electronic instructions, which may be coupled to a computer system bus. Furthermore, any computing systems referred to in the specification may include a single processor or may be architectures employing multiple processor designs for increased computing capability. Any of the steps, operations, methods or processes described herein may be performed or implemented with one or more hardware or software modules, alone or in combination with other devices. In one embodiment, a software module is implemented with a computer program product comprising a computer-readable medium containing computer program code, which can be executed by a computer processor for performing any or all of the steps, operations, methods or processes described.

Embodiments of the disclosure may also relate to a product that is produced by a computing process described herein. Such a product may comprise information resulting from a computing process, where the information is stored on a non-transitory, tangible computer readable storage medium and may include any embodiment of a computer program product or other data combination described herein.

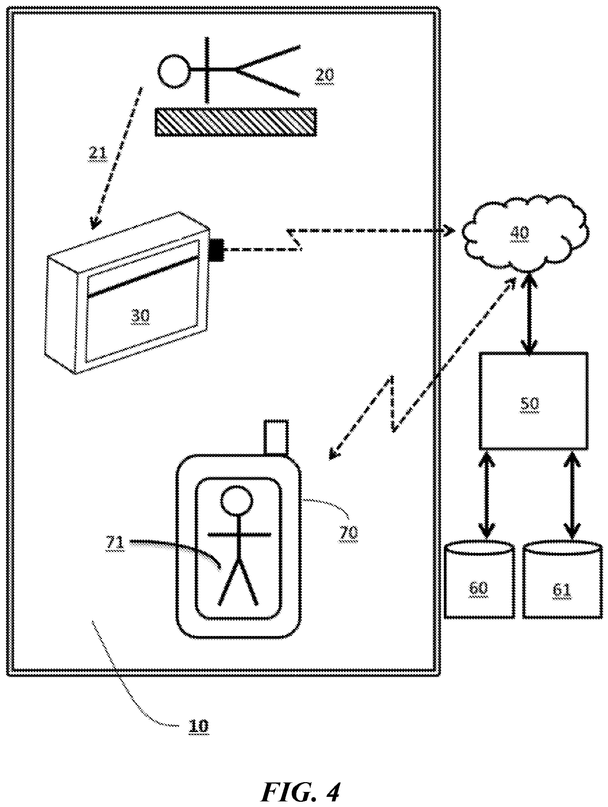

One embodiment described in Figure 4 provides a system for generation of a virtual model of drug metabolism and/or clearance (e.g. a PBPK model) in a subject 20 who may be a healthy individual or a patient in need of a therapy. The subject 20 may attend a testing facility 10 directly as shown, or may be tested from home, in the field or in a clinical setting such as a health centre, doctor's surgery or hospital. For convenience the system is shown as occurring within a testing facility 10, although it is appreciated that this is non-limiting. A liquid biopsy 21 is taken from the individual subject 20. The biopsy 21 will typically comprise a sample of blood, but may comprise any predominantly liquid sample material that comprises cell free organ mRNA. The liquid biopsy 21 is subjected to an automated RNAomics analysis 30. Once again, for convenience the analysis is shown as occurring within the testing facility 10, although it will be appreciated that remote testing via an analytics service is also a possibility. The result of the analysis 30 is communicated securely either directly or via the cloud 40 to one or more remote servers 50. The server 50 may perform computational analysis according the aforementioned methods in order to provide a quantitative output of the biopsy 21 which establishing the levels - or abundance - of xenobiotic (e.g. drug) metabolizing and/or transporting enzymes in an organ(s) of the subject 20. The server 50 may communicate with further servers 60,61 that host additional *in silico* modelling capacity (such as PBPK modelling - for example, SimCyp™ Simulator: www.certara.com) enabling generation of a more sophisticated virtual model 71 of the subject 20. Communication of this model 71 to one or more user interface/output devices 70 allows for local interrogation by a clinician or scientist with proximity to the subject 20, or even by the subject 20 themselves, where data protection and ethics regulations permit. The model 71 may be provided via the output device 70 in the form of a simulation, or via an automation interface that allows for data display and interrogation. Suitable outputs may include spreadsheets, charts, graphs, tables, figures and such like.

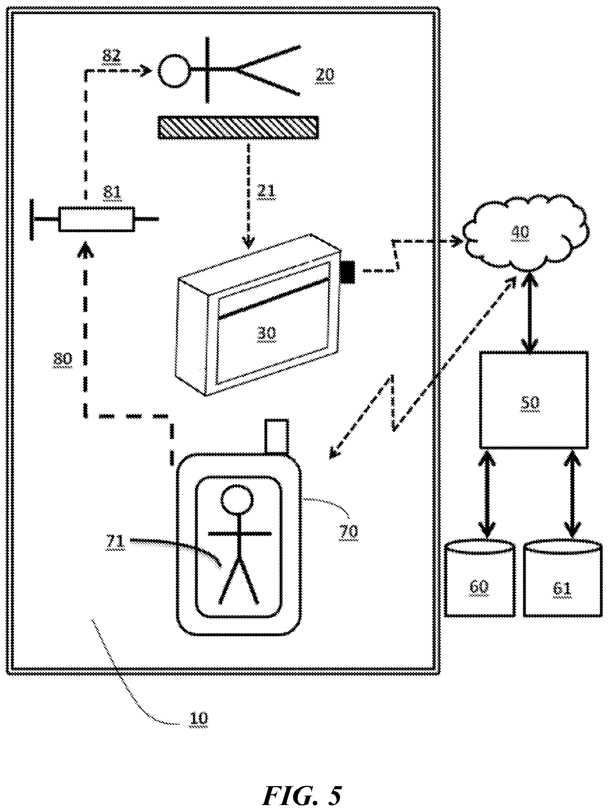

A further embodiment in Figure 5 is shown, in which the model 71 is used as an input to inform a dosage regimen 80 that is applied to a drug or other therapeutic agent, or combination of agents, 81. The drug 81 is then administered to the subject 20, in accordance with the dosage regimen 80. Optionally, additional liquid biopsy 21 may be taken following administration of the drug 81 in order to monitor a number of parameters that may be pertinent to updating of the model 71 and/or refinement of the regimen 80 going forward. The dosage regimen may be varied according to the needs of the patient as determined via the model 71, for example overtime or in relation to changing health status. Hence, in an embodiment of the disclosure there is provided a method of treating a patient over a period of time. The time may be measured in hours (e.g. at least one hour and at most 24 hours) if acute therapy is required, or over days (e.g. at least one day and at most seven days), weeks (e.g. at least one week and at most six weeks), months (e.g. at least one month and at most twelve months) or years (e.g. at least one year and at most five years or more) in the case of chronic conditions or exposure.

In embodiments of the disclosure, dosage regimens and improved personalised methods of treatment for specific classes of drugs may be optimised. Suitably, classes of drugs may include any one of the group consisting of: an anti-inflammatory drug; an anti-cancer drug; an anti-biotic; an antiviral; an anti-fungal; an analgesic; an anaesthetic; an anti-allergenic; an antidote; a hormone replacement drug; an immunosuppressive; an anti-coagulant; a cardiovascular drug; an antidepressant; an anti-diabetic; an anti-psychotic; a diuretic; a vitamin; and a sedative. In a specific embodiment of the disclosure, the dosage regime and/or improved personalised method of treatment is generated for any one or more of the products appearing on the World Health Organisation Model List of Essential Medicines (20th Edition, amended August 2017; http://www.who.int/medicines/publications/essentialmedicines/en/). In alternative embodiments, dosage regimens for any one of the pharmaceutical compounds recited above, as well as their salts, is provided for according to the methods of the present disclosure.

The invention is illustrated by the following non-limiting examples.

EXAMPLES

General Protocols

The following example provides a protocol for total RNA extraction from samples of blood that can be used to determine the levels of RNA for drug metabolizing enzymes, transporters and/or marker genes in the samples, and/or RNA for the determination of biomarkers. Methods for the isolation of total protein and quantification of enzymes and transporters are described herein for the assessment of correlation between plasma RNA and tissue protein levels.

**RNA analysis in blood**

A. Blood Samples:

Fresh peripheral venous blood may be collected from a subject and plasma isolated before further processing as described below. If required, peripheral blood mononuclear cells (PBMCs), including B and T lymphocytes, may be isolated using Ficoll-Paque PLUS (GE Healthcare Life Sciences).

Isolated plasma is stored frozen -80°C until used for cell free RNA (cfRNA) isolation and measurement. Isolation of circulating or exosomal RNA can be done using a suitable RNA extraction kit such as the Qiagen QIAamp Circulating Nucleic Acid Kit as per the manufacturer's instructions (Qiagen, Hilden, Germany). Total nucleic acid is collected by such kits, and DNA is removed using a suitable kit such as the Qiagen RNase-free DNase Set or Ambion Turbo DNA-free Kit (Life Technologies, Carlsbad, California, USA). Eluted RNA, after DNA removal, is then detected as a quality control using a suitable total nucleic acid assessment technique such as the Agilent RNA pico Kit on Bioanalyzer equipment (Agilent Technologies, Eugene, Oregon, USA). RNA of sufficient quality is then stored for subsequent quantification.

B. Reverse Transcription-PCR and Gene Sequencing:

RNA (5-10 ng) may be reverse-transcribed using M-MLV Reverse Transcriptase (Invitrogen, Life Technologies, Inc.). Samples are amplified with PCR in a final reaction volume of 25 $\mu$l containing 2.5 $\mu$l of 10 times buffer, 0.1 $\mu$l of 10 mM dNTPs, 10 pmoles of each primer and 0.5 units of Taq DNA Polymerase. To confirm the presence and integrity of the cDNA template, the housekeeping gene, GAPDH, is amplified for each sample using primers GAPDH-5 (5'-ACCACAGTCC ATGCCATCAC-3'; SEQ ID NO: 1) and GAPDH-3 (5'-TCCACCACCCTGTTGCTGTA-3'; SEQ ID NO: 2). Conditions may be as follows: an initial denaturation step for 5 minutes at 94°C, then 50 seconds at 94 °C, 45 seconds at 55 °C, and 1 min at 72 °C for 30 cycles, followed by an elongation step for 10 minutes at 72 °C.

The cDNA obtained from the extracted total RNA may be analysed further, such as via a DNA microarray, in order to determine the identities and expression levels of genes expressed within the PBMCs and the tissue biopsy samples. Alternatively, reverse transcription and amplification can be performed using a suitable genome sequencing method, such as Ampliseq (Life Technologies, ThermoFisher, Austin, TX). Up to 20,000 genes can be sequenced and several libraries (one library per sample) can be analysed in one experiment. As an example, determination of the expression of the following cytochrome P450 mono-oxygenase genes linked to drug and xenobiotic compound metabolism may be determined in both the plasma as well as in the organ samples: CYP1A2; CYP1A1; CYP1B1; CYP2A6; CYP2A7, CYP2A13; CYP2B6; CYP2C8; CYP2C9; CYP2C18; CYP2C19; CYP2D6; CYP2E1; CYP3A4; CYP3A5 and CYP3A7. Other genes which may be determined include marker genes for hepatocytes, in order to determine the degree of organ shedding in a particular individual (see Example 1, below).

The above protocol may be repeated as necessary for multiple individuals in order to generate data on expression levels of genes linked to drug and xenobiotic compound metabolism and/or the expression of organ marker genes. The data is suitable for interrogation via bioinformatics techniques to determine correlations between marker expression in circulating mRNA and expression of CYPs, for example, in the organ sample. The correlations are used to develop a virtual model of xenobiotic compound clearance that can be configured on a person by person basis in order to provide a virtual twin model of compound clearance within a given individual.

Example 1:

The following example provides a protocol for determining the degree of RNA shedding into circulation from hepatocytes in a particular subject, so establishing a robust and significant correlation function between hepatic protein levels and the corresponding plasma RNA concentrations.

*Selection of marker genes:* A set of genes expressed principally in the organ (www.proteinatlas.org) were selected and a panel of primers specific to their sequences were used to assess their expression levels in 20 blood samples from healthy individuals (2 female, age range 26-70 years) processed in three technical replicates (n = 20 × 3). These genes were selected for being specifically expressed in the organ, at significantly high levels to be considered representative of organ shedding. Among the list of these genes, a number of genes (12) which are consistently detected in plasma samples were used as organ-specific plasma markers (Table 1), which together are proposed to make up an organ/tissue shedding correction factor (SCF). This correction factor is calculated as the mean expression level of one or more of these genes in plasma, assessed using the same quantitative transcriptomic methodology (e.g. a gene sequencing technique or quantitative RT-PCR) and corrected for technical variability between samples using the total number of reads of isolated RNA. This rate of RNA shedding and the related inter-individual variability used for the normalization of expression in plasma are proposed to be a part of the correlation function with protein expression.

Table 1 shows the panel of marker genes, and the detection reproducibility of sequences of the marker RNA in plasma samples. Data are expressed as percentage of replicates where the target sequences were detected. Genes: A1BG (Alpha-1-B glycoprotein), AHSG (alpha-2-HS-glycoprotein), ALB (Albumin), APOA2 (Apolipoprotein A-II), C9 (Complement component 9), CFHR2 (Complement factor H-related 5), F2 (Coagulation factor II (thrombin)), F9 (Coagulation factor IX), HPX (Hemopexin), SPP2 (Secreted phosphoprotein 2), TF (Transferrin), SERPINC1 (Serpin peptidase inhibitor, clade C (antithrombin), member 1) and FGB (Fibrinogen beta chain).

**TABLE 1.** Reproducibility of circulating marker RNA detection in human plasma

| Genes | A1BG | AHSG | ALB | APOA 2 | CFHR 2 | F2 | F9 | HPX | SPP 2 | TF | SERPIN C1 | FGB |
|---|---|---|---|---|---|---|---|---|---|---|---|---|
| 1 ml plasma samples | 100% | 100% | 100% | 100% | 64% | 68% | 89% | 96% | 100% | 100% | 88% | 78% |
| 3 ml plasma samples | 78% | 100% | 100% | 100% | 78% | 81% | 81% | 100% | 89% | 100% | 64% | 100% |

*Correction for organ shedding:* Inter-individual variability in the expression of these markers highlights the presence of different degrees of shedding between individuals. Calculating this factor based on expression of 12 genes should assist in offsetting technical variability inherent to quantifying each of the genes individually, however it is contemplated that use of one or more of these genes or others would also be of use in carrying out the invention.

Using preliminary quantitative data on a sub-set of five samples (2 female, age range 23-57 years), inter-individual variability in SCF was estimated at 30%, calculated as percent coefficient of variation (%CV = $100 \times SD/\overline{X}$), representing up to 2-fold variability between healthy individuals. This illustrates by how much quantification of RNA coding for drug clearance proteins (or for other biomarkers) may need to be scaled in order to correct for individual variability in RNA shedding rates.

In a disease cohort, however, this level of variability increased up to 75% (n = 9), representing more than 30-fold variability between cancer patients. The average level of shedding in the cancer cohort was 25-fold higher than that in the healthy set (unpaired *t*-test, $p < 0.01$). Table 2 gives details of the cancer patients from whom matched organ and plasma samples were taken.

**TABLE 2.** Demographics of cancer patients

| Patient code | Sex | Age | Body mass index (BMI) | Diagnosis |
|---|---|---|---|---|
| 389 | Female | 52 | 31 | Metastatic Adenocarcinoma |
| 493 | Female | 50 | - | Metastatic adenocarcinoma |
| 590 | Male | 98 | 32 | Metastatic adenocarcinoma |
| 589 | Female | 52 | 21 | Metastatic adenocarcinoma |

(continued)

| Patient code | Sex | Age | Body mass index (BMI) | Diagnosis |
|---|---|---|---|---|
| 645 | Female | 61 | 36 | Metastatic adenocarcinoma |
| 646 | Male | 44 | 30 | Metastatic adenocarcinoma |
| 682 | Male | 60 | 33 | Metastatic adenocarcinoma |
| 756 | Male | 57 | 27 | Fibrosis, necrosis (due to carcinoma), inflammatory cell infiltration |
| 781 | Male | 57 | 31 | Metastatic adenocarcinoma |

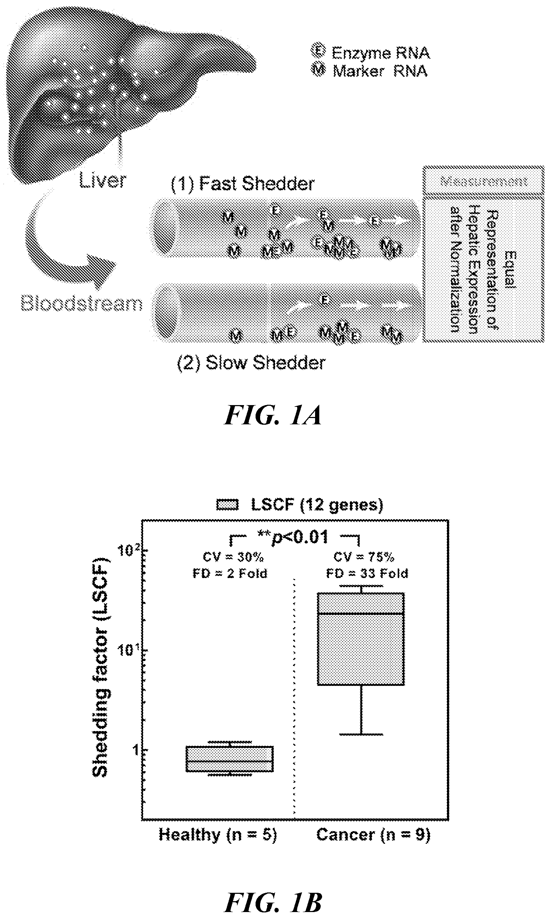

Figure 1 shows a graphical representation of the expression normalisation process. The expression levels in plasma are dependent on the level of gene expression in the organ and the degree of shedding by the organ into the bloodstream of the patient (panel A). Normalisation with organ-specific marker genes (M) provides more accurate enzyme (E) expression levels. Normalisation is carried out against a set of genes (12 in this case) specific to the organ, which make up the organ shedding correction factor (SCF) (panel B). In the example illustrated by Figure 1A, organ expression of enzyme (E) is equal and levels of shedding are different in two scenarios (fast and slow shedders). Correction for shedding using a set of markers (M) ensures correct measurement of enzyme expression. In panel B, CV is the coefficient of variation and FD is the fold difference (between maximum and minimum readings). The whiskers represent the range, the box shows the 25th and 75th percentiles and the bar shows the median. The use of an SCF based on the 12 selected genes reduces the effects of technical variability inherent to using only one gene, such as albumin (ALB), as a reference. The level of shedding in cancer patients is higher (> 25-fold) and can be more variable (30-fold) than that in healthy controls (2-fold). Without wishing to be bound by theory, the increased amount of RNA shedding (and observed larger variability) in cancer patients may result from cell death (necrosis), possibly also in response to chemotherapy. Nevertheless, the identification of this phenomenon may permit correction and accommodation within models generated by the methods of the disclosure.

Therefore, due to the presence of different levels of RNA shedding amongst patients, the proposed assessment should take into account this level of variability, with the proposed correction being applied to enzyme expression levels in plasma as follows (e.g. using 12 markers).

$$SCF = \sum_{i=1}^{12} [cfRNA]_{Marker_i} \Big/ \left( 12 \times [cfRNA]_{\text{TOTAL}} \right)$$

$$\left( [cfRNA]_{Enzyme} \right)_{Normalized} = 10^6 \cdot [cfRNA]_{Enzyme} / SCF$$

$[cfRNA]_{TOTAL}$ is the total RNA reads in a library generated from a plasma sample. The outcome should be a normalized reading for each enzyme expressed out of a million reads in a plasma sample of specified volume (1-5 ml).

Example 2

**Quantification of drug metabolizing enzymes in tissue samples**

Knowledge of the abundance of drug-metabolizing enzymes is essential for extrapolation of information on metabolic clearance (expressed per unit of enzyme) obtained from *in vitro* studies. Drug development requires optimization of pharmacokinetic properties, frequently based on prediction of *in vivo* behaviour from *in vitro* measurements. The absolute abundance level of hepatic drug-metabolizing enzymes can be determined by extrapolation of metabolism rates determined from recombinantly expressed enzymes to *in vivo* drug clearance, the so called IVIVE approach.

The quantification concatemer (QconCAT) technique was developed to quantify several proteins simultaneously in a sample and can be applied to drug-metabolizing enzymes and transporters to determine abundance levels in any given organ sample. The method involves an artificial protein comprising concatenated proteotypic signature peptides for a targeted set of proteins. The QconCAT is expressed in *Escherichia coli* in an isotopically enriched medium (Beynon et al., (2005) Nat Methods 2: 587-589), then spiked into a protein mixture, where, on proteolytic digestion, it yields isotopically labelled standard peptides for each of the targeted proteins, in equimolar concentration (Simpson and Beynon, (2012) Anal Bioanal Chem 404:977-989). QconCATs for drug metabolizing enzymes (MetCAT) and transporters (TransCAT) have been constructed and are described previously (Russell et al., (2013) J Proteome Res 12:5934-5942).

Once optimized, the QconCAT allows the full range of cytochrome P450 (CYP), transferase enzymes and/or transporters to be measured simultaneously in the same samples. Simultaneous measurement of the drug clearance enzymes and transporters facilitates creation of realistic virtual simulations as part of physiologically based pharmacokinetic-IVIVE models. In particular, expression and activity of drug-metabolizing enzymes and transporters can be correlated and correlations affecting drug absorption, distribution, metabolism, and excretion as well as absolute values are used to inform creation of *in silico* models.

Human organ samples obtained from an individual may be used to determine the levels of enzymes and transporters by quantitative techniques, such as Western blot, ELISA or LC-MS proteomics. A biopsy of organ tissue is taken from the same subject who supplied the blood sample, to provide a matched control. The tissue is physically homogenized using either a manual device (such as a ground glass Ten Broeck tissue grinder) or a mechanical/powered tissue homogenizer (e.g. a Tekmar Tissuemizer) in the presence of an appropriate extraction buffer in order to obtain a homogeneous suspension.

Differential centrifugation may be used to extract relevant fractions that can be analysed to measure protein or RNA, including homogenates, S9 fractions, cytosols and crude/microsomal membrane fractions. Transporter proteins may be measured in either homogenates or exosomal/microsomal/crude/plasma membrane fractions, CYPs and UGTs are measured in microsomal membranes and other enzymes and transferases are measured in cytosolic fractions, membrane fractions or homogenates.

The MetCAT and TransCAT standards have previously been described (Russell et al., 2013, see above). The tryptic peptides making up the QconCAT may be selected, two each from 15 cytochrome P450 (CYP) and 10 UDP-glucuronosyltransferase (UGT) enzymes (CYP1A2, CYP2A6, CYP2B6, CYP2C8, CYP2C9, CYP2C18, CYP2C19, CYP2D6, CYP2E1, CYP3A4, CYP3A5, CYP3A7, CYP3A43, CYP2J2, CYP4F2 and UGT1A1, UGT1A3, UGT1A4, UGT1A6, UGT1A9, UGT2B4, UGT2B7, UGT2B10, UGT2B11, UGT2B15). Peptides unique to subfamilies CYP1A, CYP2A, CYP2B, CYP3A, and UGT1A may be also included to enable aggregate quantification of each subfamily. The TransCAT comprises a set of transporters from two families (ABCB1, ABCB11, ABCB4, ABCC2, ABCC3, ABCC4, ABCC6, ABCG2, and SLC22A1, SLC22A3, SLC22A5, SLC22A7, SLC22A9, SLC47A1, SLCO1A2, SLCO1B1, SLCO1B3, SLCO2B1, SLCO4C1, SLC51A, SLC51B). Finally, Glu-fibrinopeptide B (SEQ ID NO: 3 EGVNDNEEGFFSAR), or a suitable analog (SEQ ID NO: 4 GGVNDNEEGFFSAR or SEQ ID NO: 5 GVNDNEEGFFSAR) can be included to enable quantification of the QconCAT, hereafter referred to as NNOP (non-naturally occurring peptide). All the required peptides are concatenated together in the MetCAT/TransCAT protein and are released on proteolytic digestion using lysyl endopeptidase and/or trypsin.

Sample and Standard Preparation

Total membrane or microsomal protein concentration may be determined in triplicate using a colorimetric assay, such as the Lowry, Bradford or BCA assay. Sample preparation may follow a gel-based (Achour et al. 2014, Drug Metab. Dispos. 42, 500-510), solution-based (Harwood et al., 2015, Pharm. Biomed. Anal. 110, 27-33) or filter-aided method (Wiśniewski et al., 2009, Nat. Methods 6, 359-362). Sample and QconCAT are co-prepared in a suitable buffer, such as 50 mM ammonium bicarbonate (~pH 8.0), normally with reduction (for example, with dithiothreitol) and alkylation (for example, with iodoacetamide) of protein disulfide bridges. Protein digestion strategies use different enzymes, mainly mammalian or recombinant trypsin. Multi-enzyme digestion strategies should afford better digestion efficiency. Protein is digested in a suitable buffer (e.g. 50 mM ammonium bicarbonate), firstly with lysyl endopeptidase (1-2% w/w) at 30°C for 3-4 hours, followed by trypsin (1-5% w/w) at 37°C for 10-18 hours. Peptide desalting, cleaning and collection is carried out. Sample volume can be reduced to a suitable volume (10-50 μl) using a vacuum concentrator and volume can be adjusted with buffer, and stored at -20°C.

Mass Spectrometric Analysis of QconCAT-Sample Mixtures

Samples are analysed by LC-MS/MS using a suitable LC-MS/MS (nano-HPLC system (e.g. nano-Acquity nanoUPLC system, Waters, UK) coupled to a triple quadruple mass spectrometer (e.g. a TSQ Vantage triple quadrupole mass spectrometer, ThermoScientific, Pittsburgh, PA; or 6500 QTRAP, SCIEX, Framingham, MA). Multiple reaction monitoring assays are designed and managed using suitable software (e.g. Skyline, MacCoss Laboratory Software, University of Washington, Seattle, WA) and data acquired by software used for operating the mass spectrometer (e.g. Xcalibur, Thermo Fisher; or Analyst, SCIEX). Samples (1-10 μl) are injected either directly onto an analytical column or onto a trapping column connected to an analytical column at a nanoflow rate using a suitable low-high acetonitrile gradient. Data are acquired for both native (unlabeled) and standard ($^{13}$C/$^{15}$N stable isotope labeled) peptides.

Data Analysis of the Measured Enzyme/Transporter Abundance

Calculation of Enzyme/Transporter Abundance Values

The abundance of each enzyme in the sample may be calculated with the following equation:

$$[\text{Protein}] = [NNOP] \times F_v \times \frac{R_{\text{NNOP}_{\frac{H}{L}}} \times R_{\text{Peptide}_{\frac{L}{H}}}}{\text{Protein Mass}}$$

where [Protein] is the estimated protein abundance in starting microsomal/crude membrane material, based on the surrogate peptide abundance estimate, measured in units of pmol mg$^{-1}$ protein. [NNOP] is the concentration of the light NNOP in the assay mix in fmol $\mu$l$^{-1}$ and $R_{\text{NNOP}_{\frac{H}{L}}}$ the ratio of heavy, QconCAT-derived NNOP to light (standard of known concentration) NNOP; together these terms quantify the QconCAT equimolar concentration. $R_{\text{Peptide}_{\frac{L}{H}}}$ is the ratio of light (sample-derived) analytical peptide to heavy (QconCAT-derived) peptide, combined with previous terms to give peptide concentration in the assay mix in fmol $\mu$l$^{-1}$. $F_v$ is the dilution factor of the sample in relation to the volume of the mixture, and the term containing it converts the peptide concentration in the assay mix to the amount in fmol in the digest. Protein Mass is the microsomal protein content corresponding to the amount of material used prior to digestion expressed in $\mu$g, the term containing it relates abundance (i.e. protein level) in the digest to abundance in microsomal protein in pmol mg$^{-1}$. The ratios $R_{\text{NNOP}_{\frac{H}{L}}}$ and $R_{\text{Peptide}_{\frac{L}{H}}}$ and are corrected for isotope incorporation efficiency prior to use in this equation.

Statistical Analysis of Enzyme/Transporter Abundance Values

Means, standard deviations, and coefficients of variation of abundances may be calculated. Normality of distribution of the abundance measurements of each enzyme/transporter can be tested according to suitable statistical tests (e.g. Kolmogorov-Smirnov, D'Agostino-Pearson, Shapiro-Wilk). The abundances can be further analysed with reference to genotype, age, ethnicity, gender, smoking, alcohol use and other associated lifestyle or genetic factors. Linear regression and correlation analysis may be carried out to test linearity and rank order relationship between the RNA expression levels in plasma and protein abundance levels in organ tissue.

Example 3

In order to determine that levels of circulating RNA for drug clearance enzymes which have been corrected for baseline shedding using an SCF as described in Example 1 above can be used to accurately estimate protein levels of the same enzymes in the organ, it is necessary to find tissue levels of these enzymes and compare them to RNA levels from the same subjects, for which the following protocol may be used.

*Organ tissue processing*

Differential centrifugation is used to isolate microsomal/crude membrane fractions. Loss due to fractionation is estimated using NADPH cytochrome P450 reductase activity (protein marker for the endoplasmic reticulum), and activity ratios allow recovery to be estimated and MPPGL (microsomal protein per gram organ) values to be calculated. Literature values for fractionation loss and MPPGL values may be found in the art (Barter Z., et al., (2007) Current Drug Metabolism; 8: 33-45). Protein recovery from homogenates was consistent across organ samples (n = 9).

*Cytochrome P450 abundance data*

The abundance data is measured in units of pmol per mg microsomal protein and thereafter converted to fmol per $\mu$g organ tissue using the MPPGL scaler (for example, CYP3A4, Table 3).
For comparison, reported values of cytochrome P450 enzymes may be found in (Achour B., et al., (2014) Drug Metabolism and Disposition; 42: 1349-1356).

**TABLE 3.** CYP3A4 organ abundance data and scaling up to tissue levels using the MPPGL scalar

| Patient ID | CYP3A4 abundance (pmol mg$^{-1}$ microsomal protein) | MPPGL (mg protein g$^{-1}$ organ) | CYP3A4 tissue level (fmol $\mu$g$^{-1}$ organ tissue) |
|---|---|---|---|
| **389** | 50.0 | 9.3 | 0.47 |

(continued)

| Patient ID | CYP3A4 abundance (pmol mg$^{-1}$ microsomal protein) | MPPGL (mg protein g$^{-1}$ organ) | CYP3A4 tissue level (fmol $\mu$g$^{-1}$ organ tissue) |
|---|---|---|---|
| 493 | 174.1 | 17.1 | 2.97 |
| 589 | 332.8 | 23.2 | 7.72 |
| 590 | 188.1 | 20.2 | 3.80 |
| 645 | 154.8 | 17.7 | 2.73 |
| 646 | 203.4 | 15.8 | 3.22 |
| 682 | 29.2 | 32.3 | 0.94 |
| 756 | 45.8 | 42.6 | 1.95 |
| 781 | 212.8 | 19.0 | 4.05 |
| Mean | 154.6 | 21.9 | 3.1 |
| Standard deviation (SD) | 98.4 | 9.9 | 2.1 |
| Coefficient of variation (CV) | 63.7% | 45.2% | 68.4% |

*Correlation of hepatic cytochrome P450 protein abundance and circulating mRNA in plasma*

Correlation analysis is performed for hepatic protein levels and plasma mRNA levels reported above the detection limit. The correlation can be compared with and without correcting the RNA data with the SCF, as shown in Example 1.

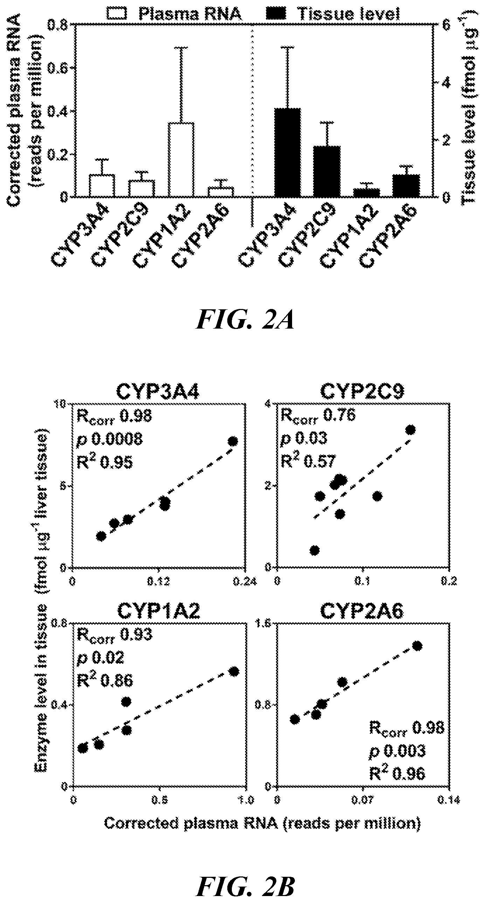

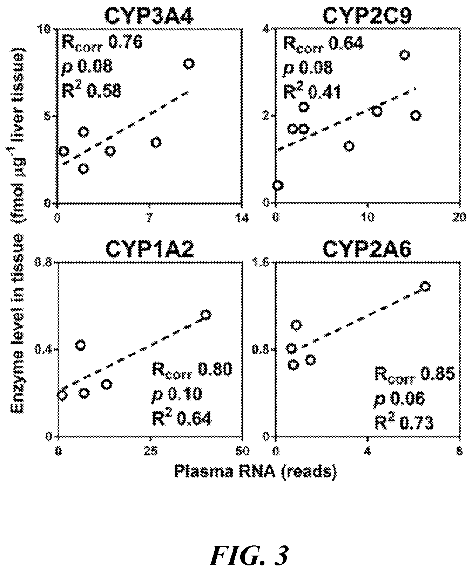

In this way, a correlation between circulating RNA and hepatic cytochrome P450 protein levels in matched samples can be found. An advantage of this protocol is that hepatic protein abundance is a much better indication of hepatic cytochrome P450 activity levels *in vivo* than are simple estimations based upon expected mRNA expression levels in the organ. Figure 2 shows levels of four enzymes (CYP3A4, CYP2C9 and CYP1A2 and CYP2A6) in organ tissue and corresponding levels of their RNA in plasma (Panel A), highlighting correlation between tissue levels and circulating RNA (Panel B). Correlation is lost when organ shedding is not considered (Figure 3). $R_{corr}$ is Pearson's correlation coefficient, $R^2$ is the coefficient of linear regression and *p* is the correlation probability.

Hence, the experiments confirm that determination of the amounts of circulating plasma mRNA can be used to identify the relative abundance of a plurality of hepatic proteins that control xenobiotic compound clearance, and that such identification is improved when the mRNA levels are controlled using an SCF indicating a baseline level of mRNA shedding in the individual. Table 4 shows examples of such determination for four specific drug clearance enzymes.

**TABLE 4.** Regression analysis for estimating tissue abundance from circulating RNA measurements

| Enzyme | Equation | $R^2$ | Number of subjects |
|---|---|---|---|
| CYP3A4 | $[CYP3A4]_{tissue} = 29.68 \times [CYP3A4]_{plasma} + 0.62$ | 0.95 | 6 |
| CYP2C9 | $[CYP2C9]_{tissue} = 17.16 \times [CYP2C9]_{plasma} + 0.46$ | 0.57 | 8 |
| CYP1A2 | $[CYP1A2]_{tissue} = 0.43 \times [CYP1A2]_{plasma} + 0.18$ | 0.86 | 5 |
| CYP2A6 | $[CYP2A6]_{tissue} = 7.54 \times [CYP2A6]_{plasma} + 0.54$ | 0.96 | 5 |

The approach taken in the present invention allows for the creation of *in silico* models that will permit the more accurate prediction of the speed of clearance of particular compounds, typically drugs or toxins, in an individual. In the absence of the shedding correction the results are highly variable between individuals rendering liquid biopsy analysis impractical and inaccurate.

**Claims**

1.   A method for identifying clearance and/or metabolic capacity of an individual subject for a specified xenobiotic compound, the method comprising:

(1) identifying at least one protein responsible for the clearance of the xenobiotic compound in a human or animal;
(2) quantifying an amount of a first cell free RNA (cfRNA) present in a liquid biopsy obtained from the individual subject, wherein the first cfRNA is derived from an organ within the body of the subject and wherein the first cfRNA codes for the at least one protein responsible for the clearance of the xenobiotic compound, the quantification comprising:

isolating total cell free RNA (cfRNA$_{TOTAL}$) from the liquid biopsy;
analysing the isolated cfRNA$_{TOTAL}$ in order to determine an amount of the first cfRNA present within the cfRNA$_{TOTAL}$; and
performing a normalizing function on the amount of the first cfRNA present against an RNA organ Shedding Correction Factor (SCF) that is determined for the subject by;

(i) performing an analysis of the cfRNA$_{TOTAL}$ in order to quantify an amount of mRNA present within the cfRNA$_{TOTAL}$ that corresponds to each of two or more marker genes, wherein a marker gene is defined as a gene that is expressed principally and consistently in the organ; and
(ii) determining SCF as the mean concentration of mRNA of the each of two or more marker genes present within the cfRNA$_{TOTAL}$;

(3) identifying the abundance of the at least one protein responsible for the clearance of the xenobiotic compound within an organ of the subject by comparison of the amount of first cfRNA encoding the at least one protein responsible for the clearance of the xenobiotic compound with an abundance curve, wherein the abundance curve is generated by comparison of matched samples comprising a cfRNA level from a liquid biopsy that codes for the at least one protein responsible for the clearance of the xenobiotic compound and the corresponding level of the protein responsible for the clearance of the xenobiotic compound from a tissue biopsy both taken from the same reference individual; and
(4) identifying the clearance capacity of the individual subject based upon the abundance of the protein responsible for the clearance of the xenobiotic compound within the organ of the subject.

2. The method of claim 1, wherein the organ is selected from the group consisting of: the liver; the kidney; the gut; the brain; and the pancreas; optionally
wherein the organ is the liver.

3. The method of any one of claims 1 to 2, wherein the protein responsible for the clearance of the xenobiotic compound is selected from the group consisting of: a xenobiotic metabolising enzyme; and a protein responsible for the transport of xenobiotics.

4. The method of claim 3, wherein the xenobiotic metabolising enzyme comprises a cytochrome P450 monooxygenase (CYP) protein; optionally
wherein CYP is selected from one of the group consisting of: CYP1A1; CYP1A2; CYP1B1; CYP2A6; CYP2A7; CYP2A13; CYP2B6; CYP2C8; CYP2C9; CYP2C18; CYP2C19; CYP2D6; CYP2E1; CYP3A4; CYP3A5; and CYP3A7.

5. The method of claim 3, wherein the xenobiotic metabolising enzyme comprises a transferase selected from one of the group consisting of: a methyltransferase; a sulfotransferase; an N-acetyltransferase; a glucuronosyltransferase including, but not limited to, one or more of the group consisting of UGT1A1, UGT1A3, UGT1A4, UGT1A6, UGT1A9, UGT2B4, UGT2B7, and UGT2B15; a glutathione-S-transferase; and a choline acetyl transferase.

6. The method of claim 3, wherein the protein responsible for the transport of xenobiotics is an ATP-binding cassette (ABC) transporter; optionally
wherein the protein responsible for the transport of xenobiotics is a solute carrier (SLC) transporter.

7. The method of any one of claims 1 to 6, wherein the SCF is determined for the subject by isolating cfRNA$_{TOTAL}$ from the liquid biopsy obtained from the subject, performing an analysis of the cfRNA$_{TOTAL}$ in order to quantify an amount of two or more marker genes mRNAs present, designated as [cfRNA]$_{Marker}$, wherein a marker gene is defined as a gene that is expressed principally and consistently in the organ and at a high level; and determining the SCF according to the formula A:

$$SCF = \sum_{i=1}^{N}[cfRNA]_{Marker_i}/(N \times [cfRNA]_{TOTAL}) \qquad \textbf{A}$$

where N is equal to the number of marker genes quantified; optionally
wherein at least three marker genes are selected in order to determine the SCF.

**Patentansprüche**

1. Verfahren zur Identifizierung der Clearance und/oder der metabolischen Kapazität eines individuellen Subjekts für eine bestimmte xenobiotische Verbindung, wobei das Verfahren umfasst:

   (1) Identifizieren mindestens eines Proteins, das für die Clearance der xenobiotischen Verbindung in einem Menschen oder Tier verantwortlich ist;
   (2) Quantifizieren einer Menge einer ersten zellfreien RNA (cfRNA), die in einer von dem individuellen Subjekt erhaltenen Flüssigbiopsie vorhanden ist, wobei die erste cfRNA von einem Organ innerhalb des Körpers des Subjekts abgeleitet ist und wobei die erste cfRNA für das mindestens eine Protein codiert, das für die Clearance der xenobiotischen Verbindung verantwortlich ist, wobei die Quantifizierung umfasst:

   Isolieren der gesamten zellfreien RNA (cfRNA$_{TOTAL}$) aus der Flüssigbiopsie;
   Analysieren der isolierten cfRNA$_{TOTAL}$, um eine Menge der ersten cfRNA zu bestimmen, die in der cfRNA$_{TOTAL}$ vorhanden ist; und
   Durchführen einer Normalisierungsfunktion an der Menge der vorhandenen ersten cfRNA gegen einen RNA-Organ-Shedding-Korrekturfaktor (SCF), der für das Subjekt bestimmt wird durch:

   (i) Durchführen einer Analyse der cfRNA$_{TOTAL}$, um eine Menge an mRNA zu quantifizieren, die in der cfRNA$_{TOTAL}$ vorhanden ist und jedem von zwei oder mehr Markergenen entspricht, wobei ein Markergen als ein Gen definiert ist, das hauptsächlich und beständig in dem Organ exprimiert wird; und
   (ii) Bestimmen des SCF als mittlere Konzentration der mRNA von jedem von zwei oder mehr Markergenen in der cfRNA$_{TOTAL}$;

   (3) Identifizieren der Abundanz des mindestens einen Proteins, das für die Clearance der xenobiotischen Verbindung innerhalb eines Organs des Subjekts verantwortlich ist, durch Vergleich der Menge der ersten cfRNA, die für das mindestens eine Protein codiert, das für die Clearance der xenobiotischen Verbindung verantwortlich ist, mit einer Abundanzkurve, wobei die Abundanzkurve erzeugt wird durch Vergleich von übereinstimmenden Proben umfassend einen cfRNA-Spiegel aus einer Flüssigbiopsie, der für das mindestens eine Protein codiert, das für die Clearance der xenobiotischen Verbindung verantwortlich ist, und den entsprechenden Spiegel des Proteins, das für die Clearance der xenobiotischen Verbindung verantwortlich ist, aus einer Gewebebiopsie, die beide demselben Referenzindividuum entnommen wurden; und
   (4) Identifizieren der Clearance-Kapazität des individuellen Subjekts basierend auf der Abundanz des Proteins, das für die Clearance der xenobiotischen Verbindung innerhalb des Organs des Subjekts verantwortlich ist.

2. Verfahren nach Anspruch 1, wobei das Organ ausgewählt ist aus der Gruppe bestehend aus: der Leber; der Niere; dem Darm; dem Gehirn; und der Bauchspeicheldrüse; optional
   wobei das Organ die Leber ist.

3. Verfahren nach einem der Ansprüche 1 bis 2, wobei das Protein, das für die Clearance der xenobiotischen Verbindung verantwortlich ist, ausgewählt ist aus der Gruppe bestehend aus: einem xenobiotischen metabolisierenden Enzym und einem für den Transport von Xenobiotika verantwortlichen Protein.

4. Verfahren nach Anspruch 3, wobei das xenobiotische metabolisierende Enzym ein Cytochrom P450-Monooxygenase (CYP)-Protein umfasst, optional
   wobei CYP ausgewählt ist aus der Gruppe bestehend aus: CYP1A1; CYP1A2; CYP1B1; CYP2A6; CYP2A7; CYP2A13; CYP2B6; CYP2C8; CYP2C9; CYP2C18; CYP2C19; CYP2D6; CYP2E1; CYP3A4; CYP3A5; und CYP3A7.

5. Verfahren nach Anspruch 3, wobei das xenobiotische metabolisierende Enzym eine Transferase umfasst, die ausgewählt ist aus der Gruppe bestehend aus: einer Methyltransferase; einer Sulfotransferase; einer N-Acetyltransferase; einer Glucuronosyltransferase, einschließlich, aber nicht beschränkt auf, eine oder mehrere aus der Gruppe bestehend aus UGT1A1, UGT1A3, UGT1A4, UGT1A6, UGT1A9, UGT2B4, UGT2B7 und UGT2B15; einer Glutathion-S-Transferase; und einer Cholinacetyltransferase.

6. Verfahren nach Anspruch 3, wobei das für den Transport von Xenobiotika verantwortliche Protein ein ATP-bindender Kassetten (ABC)-Transporter ist; gegebenenfalls
   wobei das für den Transport von Xenobiotika verantwortliche Protein ein Solute-Carrier-Transporter (SLC) ist.

7. Verfahren nach einem der Ansprüche 1 bis 6, wobei der SCF für das Subjekt bestimmt wird, indem cfRNA$_{TOTAL}$ aus der von dem Subjekt erhaltenen Flüssigbiopsie isoliert wird, eine Analyse der cfRNA$_{TOTAL}$ durchgeführt wird, um eine Menge von zwei oder mehr vorhandenen Markergenen mRNAs zu quantifizieren, die als [cfRNA]$_{Marker}$ bezeichnet werden, wobei ein Markergen als ein Gen definiert ist, das hauptsächlich und beständig in dem Organ und auf einem hohen Niveau exprimiert wird; und der SCF gemäß der Formel A bestimmt wird:

$$SCF = \sum_{i=1}^{N} [cfRNA]_{Marker_i} / (N \times [cfRNA]_{TOTAL}) \qquad \textbf{A}$$

wobei N gleich der Anzahl der quantifizierten Markergene ist; optional
wobei mindestens drei Markergene ausgewählt werden, um den SCF zu bestimmen.

## Revendications

1. Procédé d'identification de la clairance et/ou de la capacité métabolique d'un individu sujet pour un composé xénobiotique spécifié, le procédé comprenant :

(1) l'identification d'au moins une protéine responsable de la clairance du composé xénobiotique chez un être humain ou un animal ;
(2) la quantification d'une quantité d'un premier ARN acellulaire (ARNcf) présent dans une biopsie liquide obtenue à partir de l'individu sujet, dans lequel le premier ARNcf est dérivé d'un organe dans le corps du sujet et dans lequel le premier ARNcf code pour l'au moins une protéine responsable de la clairance du composé xénobiotique, la quantification comprenant :

l'isolement d'ARN acellulaire total (ARNcf$_{TOTAL}$) de la biopsie liquide ;
l'analyse de l'ARNcf$_{TOTAL}$ isolé afin de déterminer une quantité du premier ARNcf présent dans l'ARNcf$_{TOTAL}$ ; et
la réalisation d'une fonction de normalisation sur la quantité du premier ARNcf présent vis-à-vis d'un facteur de correction d'excrétion (SCF) par l'organe d'ARN qui est déterminé pour le sujet par ;

(i) la réalisation d'une analyse de l'ARNcf$_{TOTAL}$ afin de quantifier une quantité d'ARNm présent dans l'ARNcf$_{TOTAL}$ qui correspond à chacun d'au moins deux gènes marqueurs, dans lequel un gène marqueur est défini comme un gène qui est exprimé principalement et régulièrement dans l'organe ; et
(ii) la détermination du SCF en tant que concentration moyenne d'ARNm de chacun des au moins deux gènes marqueurs présent dans l'ARNcf$_{TOTAL}$ ;

(3) l'identification de l'abondance de l'au moins une protéine responsable de la clairance du composé xénobiotique dans un organe du sujet par comparaison de la quantité de premier ARNcf codant pour l'au moins une protéine responsable de la clairance du composé xénobiotique avec une courbe d'abondance, dans lequel la courbe d'abondance est générée par comparaison d'échantillons mis en concordance comprenant un taux d'ARNcf issu d'une biopsie liquide qui code pour l'au moins une protéine responsable de la clairance du composé xénobiotique et du taux correspondant de la protéine responsable de la clairance du composé xénobiotique issue d'une biopsie de tissu, tous prélevés sur le même individu de référence ; et
(4) l'identification de la capacité de clairance de l'individu sujet sur la base de l'abondance de la protéine responsable de la clairance du composé xénobiotique dans l'organe du sujet.

2. Procédé selon la revendication 1, dans lequel l'organe est sélectionné dans le groupe constitué par : le foie ; le rein ; l'intestin ; le cerveau ; et le pancréas ; éventuellement
dans lequel l'organe est le foie.

3. Procédé selon l'une quelconque des revendications 1 et 2, dans lequel la protéine responsable de la clairance du composé xénobiotique est sélectionnée dans le groupe constitué par : une enzyme métabolisant le xénobiotique ; et une protéine responsable du transport des xénobiotiques.

4. Procédé selon la revendication 3, dans lequel l'enzyme métabolisant le xénobiotique comprend une protéine à monooxygénase du cytochrome P450 (CYP) ; éventuellement
dans lequel CYP est sélectionnée à partir d'un élément du groupe constitué par : CYP1A1 ; CYP1A2 ; CYP1B1 ;

CYP2A6 ; CYP2A7 ; CYP2A13 ; CYP2B6 ; CYP2C8 ; CYP2C9 ; CYP2C18 ; CYP2C19 ; CYP2D6 ; CYP2E1 ; CYP3A4 ; CYP3A5 ; et CYP3A7.

5. Procédé selon la revendication 3, dans lequel l'enzyme métabolisant le xénobiotique comprend une transférase sélectionnée à partir d'un élément du groupe constitué par : une méthyltransférase ; une sulfotransférase ; une N-acétyltransférase ; une glucuronosyltransférase notamment, mais sans s'y limiter, un ou plusieurs éléments du groupe constitué par UGT1A1, UGT1A3, UGT1A4, UGT1A6, UGT1A9, UGT2B4, UGT2B7, et UGT2B 15 ; une glutathion-S-transférase ; et une choline acétyl transférase.

6. Procédé selon la revendication 3, dans lequel la protéine responsable du transport des xénobiotiques est un transporteur à cassette liant l'ATP (ABC) ; éventuellement
dans lequel la protéine responsable du transport des xénobiotiques est un transporteur de soluté (SLC).

7. Procédé selon l'une quelconque des revendications 1 à 6, dans lequel le SFC est déterminé pour le sujet par l'isolement d'ARNcf$_{TOTAL}$ de la biopsie liquide obtenue à partir du sujet, la réalisation d'une analyse de l'ARNcf$_{TOTAL}$ afin de quantifier une quantité d'au moins deux ARNm de gènes marqueurs présents, désignés par [ARNcf]$_{Marqueur}$, dans lequel un gène marqueur est défini comme un gène qui est exprimé principalement et régulièrement dans l'organe et à un taux élevé ; et la détermination du SFC selon la formule A :

$$SCF = \sum_{t=}^{N} [ARNcf]_{Marqueur} \, / (N \times [ARNcf]_{TOTAL}) \qquad \mathbf{A}$$

où N est égal au nombre de gènes marqueurs quantifiés ; éventuellement
dans lequel au moins trois gènes marqueurs sont sélectionnés afin de déterminer le SCF.

**FIG. 1A**

**FIG. 1B**

*FIG. 2A*

*FIG. 2B*

*FIG. 3*

*FIG. 4*

*FIG. 5*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160335412 A

- WO 0200935 A, Ramanathan

### Non-patent literature cited in the description

- **ZHOU et al.** *Clin Pharmacol Ther*, July 2018, vol. 104 (1), 188-200
- **SPANAKIS** ; **MARIAS**. *In Silico Pharmacol.*, December 2014, vol. 2 (1), 2
- **EL-HEFNAWY et al.** *Clin Chem*, 2004
- **EINOLF et al.** *Clin Pharmacol Ther.*, February 2014, vol. 95 (2), 179-88
- **VAN DER HAUWAERT et al.** *Toxicology and Applied Pharmacology*, 03 September 2014, vol. 279
- **LAM et al.** *Drug Metabolism and Disposition*, December 2010, vol. 38 (12)
- **T. CORMEN** ; **C. LEISERSON** ; **R. RIVEST**. Introduction to Algorithms. The MIT Press, 2009
- **L. ERIKSSON** ; **E. JOHANSSON** ; **N. KETTANEH-WOLD** ; **J. TRYGG** ; **C. WIKSTOM** ; **S. WOLD**. Multi- and Megavariate Data Analysis. UMetrics, 2006
- **M.R. GREEN** ; **J. SAMBROOK**. Molecular Cloning: A Laboratory Manual. Cold Spring Harbor Laboratory Press, Cold Spring Harbor, 2012
- **AUSUBEL, F. M et al.** Current Protocols in Molecular Biology. John Wiley & Sons, 1995
- **B. ROE** ; **J. CRABTREE** ; **A. KAHN**. DNA Isolation and Sequencing: Essential Techniques. John Wiley & Sons, 1996
- **J. M. POLAK** ; **JAMES O'D. MCGEE**. In Situ Hybridisation: Principles and Practice. Oxford University Press, 1990
- Oligonucleotide Synthesis: A Practical Approach. IRL Press, 1984
- **D. M. J. LILLEY** ; **J. E. DAHLBERG**. Methods of Enzymology: DNA Structure Part A: Synthesis and Physical Analysis of DNA Methods in Enzymology. Academic Press, 1992

- **LEHNINGER, A. L**. Biochemistry. Worth Publishers, 1975, 71-92
- **ROBERTS** ; **VELLACCIO**. The Peptides: Analysis, Synthesis, Biology, Gross and Meiehofer. Academic Press, Inc, 1983, vol. 5, 341
- **ZANGER** ; **SCHWAB**. *Pharmacology & Therapeutics*, 2013, vol. 138, 103-141
- **WATARI et al.** *Biol. Pharm. Bull.*, 2019, vol. 42, 348-353
- **JAMEI M** ; **MARCINIAK S** ; **FENG K** ; **BARNETT A** ; **TUCKER G**. *Rostami-Hodjegan A, Expert Opin Drug Metab Toxicol*, February 2009, vol. 5 (2), 211-23
- **AUGUST 2017**. *World Health Organisation Model List of Essential Medicines*, http://www.who.int/medicines/publications/essentialmedicines/en
- **BEYNON et al.** *Nat Methods*, 2005, vol. 2, 587-589
- **SIMPSON** ; **BEYNON**. *Anal Bioanal Chem*, 2012, vol. 404, 977-989
- **RUSSELL et al.** *J Proteome Res*, 2013, vol. 12, 5934-5942
- **ACHOUR et al.** *Drug Metab. Dispos.*, 2014, vol. 42, 500-510
- **HARWOOD et al.** *Pharm. Biomed. Anal.*, 2015, vol. 110, 27-33
- **WIŚNIEWSKI et al.** *Nat. Methods*, 2009, vol. 6, 359-362
- **BARTER Z et al.** *Current Drug Metabolism*, 2007, vol. 8, 33-45
- **ACHOUR B et al.** *Drug Metabolism and Disposition*, 2014, vol. 42, 1349-1356